# EUROPEAN PATENT SPECIFICATION

(11) **EP 1 032 654 B1**
(45) Date of publication and mention of the grant of the patent: **21.01.2009**
(21) Application number: 98958217.6
(22) Date of filing: 26.11.1998
(51) Int. Cl.: C12N 9/34, C12N 1/14, C12P 19/20

(54) **THERMOSTABLE GLUCOAMYLASE**
THERMOSTABILE GLUKOAMYLASE
GLUCOAMYLASE THERMOSTABLE

(30) Priority: 26.11.1997 US 979673; 30.12.1997 DK 155797; 30.06.1998 US 107657; 10.07.1998 DK 92598
(43) Date of publication of application: 06.09.2000
(73) Proprietor: Novozymes A/S, 2880 Bagsvaerd (DK)
(72) Inventor: NIELSEN, Bjarne, Roenfeldt, 2880 Bagsvaerd (DK); NIELSEN, Ruby, Ilum, 2880 Bagsvaerd (DK); LEHMBECK, Jan, 2880 Bagsvaerd (DK)
(86) International application number: PCT/DK1998/000520
(87) International publication number: WO 1999/028448

(56) References cited:
- EP-A2- 0 135 138
- EP-A2- 0 255 124
- WO-A1-86/01831
- US-A- 4 247 637
- US-A- 4 587 215
- PATENT ABSTRACTS OF JAPAN, Vol. 11, No. 184, (C-427); & JP 62006678 A (TAX ADM AGENCY) 13 January 1987.
- DIALOG INFORMATION SERVICES, File 351, DERWENT WPI, Dialog Accession No. 007454391, WPI Accession No. 88-088325/198813, HITACHI LTD., "Aerobic Clostridium sp. G-0005 Bacterium - Which Produces Thermo-Resistant and Acid-Resistant Glucoamylase"; & JP 63039577 A (20-02-88) Week 198813 B.
- EMBL, DATABASE/GENBANK/DDBJ, Accession No. D01035, HATA Y. et al., "The Glucoamylase cDNA from Aspergillus Oryzae: Its Cloning, Nucleotide", AOGLA 09 Oct. 1993; & AGRIC. BIOL. CHEM., 55, (1991), pages 941-949, SEQ ID No. 7, 62% Homology.
- EMBL, DATABASE/GENBANK/DDBJ, Accession No. P40212, CETUS CORP., "Sequence Encoded by A. Awamori Glucoamylase Genomic Region", GENESEQ P40212, 09 Jan. 1992; & WO 8402921 A.
- EMBL, DATABASE/GENBANK/DDBJ, Accession No. E03645, HATA Y. et al., "Novel Gene, Vector, Transformant Using the Same and Use of the Transformant", Empatent:E03645, 08 Oct. 1997; & JP 4148683 A, 21 May 1992.
- EMBL, DATABASE/GENBANK/DDBJ, Accession No. P36914, HATA Y. et al., "Glucoamylase Precursor (EC3.2.1.3) (Glucan 1,4-alpha-Glucosidase)(1,4-alpha-D-Glucan Glucohydrolase), Swissprot", AMYG ASPOR, 01 Jun. 1994; & AGRIC. BIOL. CHEM., 55, (1991), pages 941-949, SEQ ID No. 3, 85,7% Homology.
- EMBL, DATABASE/GENBANK/DDBJ, Accession No. Q04731, JOZO SHIGEN KENKYUS, "cDNA Sequence from mRNA of Glucoamylase Gene", Geneseq Q04731, 12 Oct. 1990; & JP 2119779 A (07-05-90).
- EMBL, DATABASE/GENBANK/DDBJ, Accession No. L15383, VENTURA L. et al., "Molecular Cloning and Transcriptional Analysis of the Aspergillus", ATGLUAMY, 15 Mar. 1994; & APPL. ENVIRON. MICROBIOL., 61, (1995), pages 399-402, SEQ ID No. 5, 88% Homology.

## Description

### FIELD OF THE INVENTION

The present invention relates to a thermostable glucoamylase suitable for, *e.g.*, starch conversion, *e.g.*, for producing glucose from starch. The present invention also relates to the use of said thermostable glucoamylase in various processes, in particular in the saccharification step in starch convention processes.

### BACKGROUND OF THE INVENTION

Glucoamylases, (1,4-α-D-glucan glucohydrolase, EC 3.2.1.3) are enzymes which catalyze the release of D-glucose from the non-reducing ends of starch or related oligo- and polysaccharide molecules.

Glucoamylases are produced by several filamentous fungi and yeasts, including *Aspergillus niger* and *Aspergillus awamori*.

Commercially, the glucoamylases are used to convert corn starch which is already partially hydrolyzed by an α-amylase to glucose. The glucose may further be converted by glucose isomerase to a mixture composed almost equally of glucose and "fructose. This mixture, or the mixture further enriched with fructose, is the commonly used high fructose corn syrup commercialized throughout the world. This syrup is the world's largest tonnage product produced by an enzymatic process. The three enzymes involved in the conversion of starch to fructose are among the most important industrial enzymes produced.

One of the main problems existing with regard to the commercial use of glucoamylase in the production of high fructose corn syrup is the relatively low thermal stability of glucoamylases, such as the commercially available *Aspergillus niger* glucoamylase (*i.e.*, (sold as AMG by Novo Nordisk A/S). The commercial *Aspergillus* glucoamylase is not as thermally stable as α-amylase or glucose isomerase and it is most active and stable at lower pH's than either α-amylase or glucose isomerase. Accordingly, it must be used in a separate vessel at a lower temperature and pH.

US patent no. 4,247,637 describes a thermostable glucoamylase having a molecular weight of about 31,000 Da derived from *Talaromyces duponti* suitable for saccharifying a liquefied starch solution to a syrup. The glucoamylase is stated to retain at least about 90% of its initial glucoamylase activity when held at 70°C for 10 minutes at pH 4.5.

US patent no. 4,587,215 discloses a thermostable amyloglucosidase derived from the species *Talaromyces thermophilus* with a molecular weight of about 45,000 Da. The disclosed amyloglucosidase (or glucoamylase) loses its enzymatic activity in two distinct phases, an initial period of rapid decay followed by a period of slow decay. At 70°C (pH=5.0) the half-life for the fast decay is about 18 minutes with no measurable loss of activity within an hour in the second phase of decay.

Bunni L et al., (1989), Enzyme Microb. Technol., Vol. 11, p. 370-375. concerns production, isolation and partial characterization of an extracellular amylolytic system composed of at least one form of α-amylase and one form of an α-glucosidase produced by *Talaromyces emersonii* CBS 814.70. Only the α-amylase is isolated, purified and characterized.

### BRIEF DISCLOSURE OF THE INVENTION

The present invention is based upon the finding of a novel thermostable glucoamylase suitable for use, *e.g.,* in the saccharification step in starch conversion processes.

The terms "glucoamylase" and "AMG" are used interchangeably below.

The thermal stability of the glucoamylase of the invention is measured as T_{1/2} (half-life) using the method described in the "Materials and Methods" section below.

The inventors of the present invention have isolated, purified and characterized a thermostable glucoamylase from a strain of *Talaromyces emersonii* now deposited with the Centraalbureau voor Schimmelcultures under the number CBS 793.97.

When applied to a protein, the term "isolated" indicates that the protein is found in a condition other than its native environment. In a preferred form, the isolated protein is substantially free of other proteins, particularly other homologous proteins (*i.e.*, "homologous impurities" (see below)).

It is preferred to provide the protein in a greater than 40% pure form, more preferably greater than 60% pure form. Even more preferably it is preferred to provide the protein in a highly purified form, *i.e.*, greater than 80% pure, more preferably greater than 95% pure, and even more preferably greater than 99% pure, as determined by SDS-PAGE.

The term "isolated enzyme" may alternatively be termed "purified enzyme".

The term "homologous impurities" means any impurity (*e.g.* another polypeptide than the polypeptide of the invention) which originates from the homologous cell, from where the polypeptide of the invention is originally obtained.

The isolated glucoamylase has a very high thermal stability in comparison to prior art glucoamylases, such as the *Aspergillus* niger glucoamylase (available from Novo Nordisk A/S under the trade name AMG). The T½ (half-life) was determined to be about 120 minutes at 70°C (pH 4.5) as described in Example 2 below. The T½ of the recombinant *T. emersonii* AMG expressed in yeast was determined to be about 110 minutes as described in Example 12.

Therefore, in the first aspect the present invention relates to an isolated enzyme with glucoamylase activity, wherein the enzyme is derived from *Talaromyces emersonii,* exhibits a degree of identity of at least 80% with the amino acid sequence shown in SEQ ID NO: 7, and has a T_{1/2} (half-life) of at least 100 minutes in 50 mM NaOAc, 0.2 AGU/ml, pH 4.5, at 70°C.

In the second aspect the invention relates to a cloned DNA sequence derived from *Talaromyces emersonii* and encoding an enzyme exhibiting glucoamylase activity, which DNA sequence comprises: (a) the glucoamylase encoding part of the DNA sequence shown in SEQ ID NO: 33; (b) the DNA sequence shown in positions 649-2724 in SEQ ID NO:33 or its complementary strand; .(c) an analogue of the DNA sequence defined in (a) or (b) which is at least 80% homologous with said DNA sequence; (d) a DNA sequence which hybridizes with a double-stranded DNA probe comprising the sequence shown in 649-2724 in SEQ ID NO: 33 at medium stringency; (e) a DNA sequence which, because of the degeneracy of the genetic code, does not hybridize with the sequences of (b) or (c), but which codes for a polypeptide having exactly the same amino acid sequence as the polypeptide encoded by any of these DNA sequences.

The term "partial sequence" denotes a partial polypeptide sequence which is comprised in a longer polypeptide sequence, wherein said longer polypeptide sequence is having the activity of interest.

Further, the invention also relates to a process of converting starch or partially hydrolyzed starch into a syrup containing, *e.g.*, dextrose, said process including the step of saccharifying starch hydrolyzate in the presence of a glucoamylase of the first aspect or a glucoamylase encoded by a cloned DNA sequence of the second aspect.

In a further aspect the invention also relates a method of saccharifying a liquefied starch solution, which method comprises an enzymatic saccharification step using a glucoamylase according to any of claims 1-3 or a glucoamylase encoded by a DNA sequence of claims 4 or 5.

In still further aspects the invention relates to uses of a glucoamylase According to the first aspect or a glucoamylase encoded by a DNA sequence according to the second aspect in a starch conversion process, a continuous starch conversion process, a process for producing oligosaccharides, a process for producing specially syrups, a process for producing ethanol for fuel, a process for producing a beverage and/or in a fermentation process for producing organic compounds, such as citric acid, ascorbic acid, lysine, glutamic acid.

Finally, the invention relates to an isolated pure culture of the microorganism Talaromyces emersonii CBS 793.97 capable of producing a glycoamylase according to the first aspect or a glucoamylase encoded by a DNA sequence according to the second.

It is an object of the invention to provide a method of saccharifying a liquefied starch solution, wherein an enzymatic saccharification is carried out using a glucoamylase of the invention.

Furthermore, the invention relates to the use of a glucoamylase of the invention in a starch conversion process, such as a continuous starch conversion process. In an embodiment of the continuous starchs conversion process it includes a continuous saccharification step.

The glucoamylase of the invention may also be used in processes for producing oligosaccharides or specialty syrups.

Finally, the invention relates to an isolated pure culture of the microorganism *Talaromyces emersonii* CBS 793.97 or a mutant thereof capable of producing a glucoamylase of the invention.

### BRIEF DESCRIPTION OF THE DRAWINGS

Figure 1 shows the SDS-PAGE gel (stained with Coomassie Blue) used for determining the molecular weight (M_{w}) of the purified *Talaromyces emersonii* CBS 793.97 glucoamylase of the present invention.
   1: Standard marker,
   2: Q Sepharose pool (1. run)
   3: S Sepharose pool;
Figure 2 shows the pH activity profile of *Talaromyces emersonii* and *Aspergillus niger* glucoamylase (AMG) in 0.5% maltose at 60°C;
Figure 3 shows the temperature activity profile of the *Talaromyces emersonii* CBS 793.97 glucoamylase vs. *Aspergillus niger* glucoamylase (AMG);
Figure 4 shows the curve for determining T_{1/2} (half-life) in 50 mM NaOAc, 0.2 AGU/ml, pH 4.5, at 70°C of *Talaromyces emersonii* CBS 793.97 glucoamylase vs. *Aspergillus niger* glucoamylase (AMG);
Figure 5 shows the sequence of the *Talaromyces emersonii* AMG locus. The predicted amino acid sequence is shown below the nucleotide sequence. The four introns are shown in lower case letters. Consensus introns sequences are underlined. Putative signal and pro-peptides are double underlined and dotted underline, respectively;
Figure 6 shows an alignment/comparison of the amino acid sequences of the *A.niger* AMG (An_amg-1.pro), *A.oryzae* AMG Ao_AMG.pro), and *Talaromyces emersonii* AMG (Tal-AMG.pro). Identical amino acid residues are indicated by a *. Signal and pro peptides are underlined by a single and a double lined, respectively;
Figure 7 shows the *Aspergillus* expression cassette pCaHj483 used in Example 5;
Figure 8 shows the *Aspergillus* expression plasmid, pJal518, for the *Talaromyces emersonii* AMG gene;
Figure 9 shows the construction of *A.niger* disruption plasmid;
Figure 10 shows the SDS page gel of two transformants, JaL228#5.77 and HowB112#8.10, expressing the *Talaromyces emersonii* glucoamylase of the invention. JaL228 and HowB112 are the untransformed parent strains. MW: Promega's Protein Molecular;

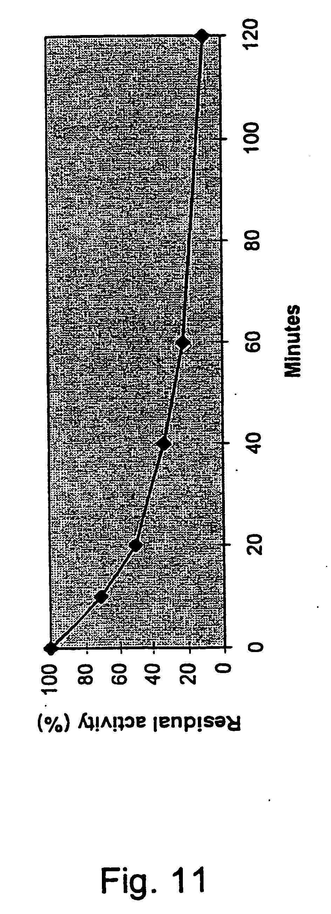

Figure 11 shows the thermal stability of the *T. emersonii* AMG produced the strain *A. niger* HowB112 determined in 50mM NaOAC, pH 4.5, 70°C, 0.2 AGU/ml (T1/2 determined to 20 minutes);
Figure 12 compares the thermal stability at 68°C of the fermentation broth of *T. emersonii* AMG expressed in yeast produced in yeast and the *A. niger* AMG;
Figure 13 shows the result of, the test for determining the thermostability of recombinant *Talaromyces emersonii* AMG produced in yeast at 70°C, pH 4.5, 0.2 AGU/ml. T1/2 was determined to about 110°C.

### DETAILED DISCLOSURE OF THE INVENTION

The present invention is based upon the finding of a novel thermostable glucoamylase suitable for use in, *e.g.*, the saccharification step in a starch conversion process.

The inventors of the present invention have isolated, purified and characterized a glucoamylase from a strain of *Talaromyces emersonii* CBS 793.97. The glucoamylase turned out to have a very high thermal stability in comparison to prior art glucoamylases.

Accordingly, in a first aspect the present invention relates to an isolated enzyme with glucoamylase activity, wherein the enzyme is derived from *Talaromyces emersonii*, exhibits a degree of identity of at least 80% with the amino acid sequence shown in SEQ ID NO: 7, and has a T_{1/2} (half-life) of at least 100 minutes in 50 mM NaoAc, 0.2 AGU/ml, pH 4.5, at 70°C.

T1/2 (half-life) of the isolated *Talaromyces emersonii* CBS 793.97 glucoamylase was determined to be about 120 minutes at 70°C as described in Example 2 below and to be about 110 minutes for the *T. emersonii* produced in yeast as described in Example 12.

The molecular weight of the isolated glucoamylase was found to be about 70 kDa determined by SDS-PAGE. Further, the pI of said enzyme was determined to be below 3.5 using isoelectrical focusing.

The isoelectric point, pI, is defined as the pH value where the enzyme molecule complex (with optionally attached metal or other ions) is neutral, *i.e.*, the sum of electrostatic charges (net electrostatic charge, NEC) on the complex is equal to zero. In this sum of course consideration of the positive or negative nature of the electrostatic charge must be taken into account.

It is expected that substantially homologous enzymes having the same advantageous properties are obtainable from other micro-organisms, especially fungal organisms such as filamentous fungi, in particular from another strain of *Talaromyces,* especially another strains of *Talaromyces emersonii.*

### The deposited micro-organism

An isolate of the filamentous fungus strain, from which the glucoamylase of the invention has been isolated, has been deposited with the Centraalbureau voor Schimmelcultures, P.O. Box 273, 3740 AG Baarn, the Netherlands, for the purposes of patent procedure on the date indicated below. CBS being an international depository under the Budapest Treaty affords permanence of the deposit in accordance with rule 9 of said treaty.
Deposit date June 2, 1997
Depositor's ref.: NN049253
CBS designation : CBS 793.97

The isolate of the filamentous fungus *Talaromyces emersonii* CBS No. 793.97 has been deposited under conditions that assure that access to the isolated fungus will be available during the pendency of this patent application to one determined by the commissioner of Patents and Trademarks to be entitled thereto under 37 C.F.R. § 1.14 and 35. U.S.C § 122. The deposit represents a substantially pure culture of the isolated fungus. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

### Talaromyces emersonii glucoamylase amino acid sequence

The inventors have sequenced the thermostable glucoamylase derived from *Talaromyces emersonii* CBS 793.97 as will be described further in the Example 3 below. According to the invention the *Talaromyces* AMG may have a Asp145Asn (or D145N) substitution (using SEQ ID NO: 7 numbering).

Therefore, the invention also relates to an isolated enzyme with glucoamylase activity comprising one or more of the partial sequences shown in SEQ ID NOS: 1-6 or the full length sequence shown in SEQ ID NO: 7 or an enzyme with glucoamylase activity being substantially homologous thereto. SEQ ID NO: 34 shows the full length sequence including the signal and pre propeptide from amino acid no. 1 to 27.

### Homology of the protein sequence

The homology between two glucoamylases is determined as the degree of identity between the two protein sequences indicating a derivation of the first sequence from the second. The homology may suitably be determined by means of computer programs known in the art such as gap provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, p. 443-453). Using gap with the following settings for polypeptide sequence comparison: gap creation penalty of 3.0 and gap extension penalty of 0.1.

According to the invention a "substantially homologous" amino acid sequence exhibits a degree of identity preferably of at least 80%, at least 90%, more preferably at least 95%, more preferably at least 97%, and most preferably at least 99% with the partial amino acid sequences shown in SEQ ID NO: 1-6 or SEQ ID NO: 7.

### The Cloned Talaromyces emersonii DNA sequence

The invention also relates to a cloned DNA sequence encoding an enzyme exhibiting glucoamylase activity of the invention, which DNA sequence comprises:
(a) the glucoamylase encoding part of the DNA sequence shown in SEQ ID NO: 33;
(b) the DNA sequence shown in positions 649-2724 in SEQ ID NO:33 or its complementary strand;
(c) an analogue of the DNA sequence defined in (a) or (b) which is at least 80% homologous with said DNA sequence;
(d) a DNA sequence which hybridizes with a double-stranded DNA probe comprising the sequence shown in 649-2724 in SEQ ID NO: 33 at low stringency;
(e) a DNA sequence which, because of the degeneracy of the genetic code, does not hybridize with the sequences of (b) or (c), but which codes for a polypeptide having exactly the same amino acid sequence as the polypeptide encoded by any of these DNA sequences; or
(g) a DNA sequence which is a fragment of the DNA sequences specified in (a), (b), (c), (d), or (e).

The mature part of the AMG of the invention is encoded by the DNA sequence in position 728-2724 of SEQ ID NO: 33. When expressing the AMG of the invention in yeast, e.g., *Saccharomyces cerevisiae* YNG318, the introns need to be cut out as described in Example 7.

### Homology of DNA sequences

The DNA sequence homology referred to above is determined as the degree of identity between two sequences indicating a derivation of the first sequence from the second. The homology may suitably be determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3, the coding region of the analogous DNA sequences referred to above exhibits a degree of identity preferably of at least 80%, more preferably at least 90%, more preferably at least 95%, more preferably at least 97% with the AMG encoding part of the DNA sequence shown in SEQ ID NO: 33 or the glucoamylase encoding part with or witout introns.

### Hybridization:

The hybridization conditions referred to above to define an analogous DNA sequence as defined in d) above which hybridizes
to a double-stranded DNA probe comprising the sequence shown in positions 649-2748 in SEQ ID NO: 33 (*i.e.*, the AMG encoding part), under at least low stringency conditions, but preferably at medium or high stringency conditions are as described in detail below.

Suitable experimental conditions for determining hybridization at low, medium, or high stringency between a nucleotide probe and a homologous DNA or RNA sequence involves presoaking of the filter containing the DNA fragments or RNA to hybridize in 5 x SSC (Sodium chloride/Sodium citrate, Sambrook et al. 1989) for 10 min, and prehybridization of the filter in a solution of 5 x SSC, 5 x Denhardt's solution (Sambrook et al. 1989), 0.5 % SDS and 100 µg/ml of denatured sonicated salmon sperm DNA (Sambrook et al. 1989), followed by hybridization in the same solution containing a concentration of 10ng/ml of a random-primed (Feinberg, A. P. and Vogelstein, B. (1983) Anal. Biochem. 132:6-13), ³²P-dCTP-labeled (specific activity > 1 x 10⁹ cpm/µg ) probe for 12 hours at about 45°C. The filter is then washed twice for 30 minutes in 2 x SSC, 0.5 % SDS at about 55°C (low stringency), more preferably at about 60°C (medium stringency) still more preferably at about 65°c (medium/high stringency), even more preferably at about 70°c (high stringency), and even more preferably at about 75°C (very high stringency).

Molecules to which the oligonucleotide probe hybridizes under these conditions are detected using a x-ray film.

### Starch conversion

The present invention provides a method of using the thermostable glucoamylase of the invention for producing glucose and the like from starch. Generally, the method includes the steps of partially hydrolyzing precursor starch in the presence of α-amylase and then further hydrolyzing the release of D-glucose from the non-reducing ends of the starch or related oligo- and polysaccharide molecules in the presence of glucoamylase by cleaving α- (1→4) and α- (1→6) glucosidic bonds.

The partial hydrolysis of the precursor starch utilizing α-amylase provides an initial breakdown of the starch molecules by hydrolyzing internal α-(1→4)-linkages. In commercial applications, the initial hydrolysis using α-amylase is run at a temperature of approximately 105°C. A very high starch concentration is processed, usually 30% to 40% solids. The initial hydrolysis is usually carried out for five minutes at this elevated temperature. The partially hydrolyzed starch can then be transferred to a second tank and incubated for approximately one hour at a temperature of 85° to 90°C to derive a dextrose equivalent (D.E.) of 10 to 15.

The step of further hydrolyzing the release of D-glucose from the non-reducing ends of the starch or related oligo- and polysaccharides molecules in the presence of glucoamylase is normally carried out in a separate tank at a reduced temperature between 30° and 60°C. Preferably the temperature of the substrate liquid is dropped to between 55° and 60°C. The pH of the solution is dropped from 6 to 6.5 to a range between 3 and 5.5. Preferably, the pH of the solution is 4 to 4.5. The glucoamylase is added to the solution and the reaction is carried out for 24-72 hours, preferably 36-48 hours.

By using a thermostable glucoamylase of the invention saccharification processes may be carried out at a higher temperature than traditional batch saccharification processes. According to the invention saccharification may be carried out at temperatures in the range from above 60-80°C, preferably 63-75°C. This applies both for traditional batch processes (described above) and for continuous saccharification processes.

Actually, continuous saccharification processes including one or more membrane separation steps, *i.e.*. filtration steps, must be carried out at temperatures of above 60°C to be able to maintain a reasonably high flux over the membrane. Therefore, a thermostable glucoamylase of the invention provides the possibility of carrying out large scale continuous saccharification processes at a fair price within and period of time acceptable for industrial saccharification processes. According to the invention the saccharification time may even be shortened.

The activity of a glucoamylase of the invention is generally substantially higher at temperatures between 60°C-80°C than at the traditionally used temperature between 30-60°C. Therefore, by increasing the temperature at which the glucoamylase operates the saccharification process may be carried out within a shorter period of time or the process may be carried out using lower enzyme dosage.

As the thermal stability of the glucoamylase of the invention is very high in comparison to, *e.g.*, the commercially available *Aspergillus niger* glucoamylase (*i.e.*, AMG) a less amount of glucoamylase needs to be added to replace the glucoamylase being inactivated during the saccharification process. More glucoamylase is maintained active during saccharification process according to the present invention. Furthermore, the risk of microbial contamination is also reduced when carrying the saccharification process at temperature above 63°C.

By using a glucoamylase with increased specific activity (measured as activity towards maltose), a lower enzyme dosage may be required in the saccharification process.

Examples of saccharification processes, ..... wherein the glucoamylase of the invention may advantageously be used include the processes described in JP 3-224493; JP 1-191693; JP 62-272987; and EP 452,238.

In a further aspect the invention relates to a method of saccharifying a liquefied starch solution, which method comprises an enzymatic saccharification step using a glucoamylase of the invention.

The glucoamylase of the invention may be used in the present inventive process in combination with an enzyme that hydrolyzes only α-(1→6)-glucosidic bonds in molecules with at least four glucosyl residues. Preferably, the glucoamylase of the invention is used in combination with pullulanase or isoamylase. The use of isoamylase and pullulanase for debranching, the molecular properties of the enzymes, and the potential use of the enzymes with glucoamylase is set forth in G.M.A. van Beynum et al., Starch Conversion Technology, Marcel Dekker, New York, 1985, 101-142.

In a further aspect the invention relates to the use of a glucoamylase of the invention in a starch conversion process.

Further, the glucoamylase of the invention may be used in a continuous starch conversion process including a continuous saccharification step.

The glucoamylase of the invention may also be used in immobilised form. This is suitable and often used for producing speciality syrups, such as maltose syrups, and further for the raffinate stream of oligosaccharides in connection with the production of fructose syrups.

The glucoamylase of the invention may also be used in a process for producing ethanol for fuel or beverage or may be used in a fermentation process for producing organic compounds, such as citric acid, ascorbic acid, lysine, glutamic acid.

### MATERIALS AND METHODS

### Material

### Enzymes:

Glucoamylase derived from the deposited filamentous fungus *Talaromyces emersonii* CBS No. 793.97 hasbeen deposited with the Centraalbureau voor Schimmelcultures, P.O. Box 273, 3740 AG Baarn, the Netherlands, for the purposes of patent procedure on the date indicated below. CBS being an international depository under the Budapest Treaty affords permanence of the deposit in accordance with rule 9 of said treaty.
Deposit date : June 2, 1997
Depositor's ref.: NN049253
CBS designation : CBS 793.97

Glucoamylase G1 derived from *Aspergillus niger* disclosed in Boel et al. (1984), EMBO J. 3 (5), 1097-1102, available from Novo Nordisk and shown in SEQ ID NO: 9.

### Strains:

JaL228; Construction of this strain is described in WO98/12300 SMO110; Construction of this strain is described in Example 6 Yeast Strain: *Saccharomyces cerevisiae* YNG318: MATa leu2-D2 ura3-52 his4-539 pep4-D1 [cir+].

### Genes:

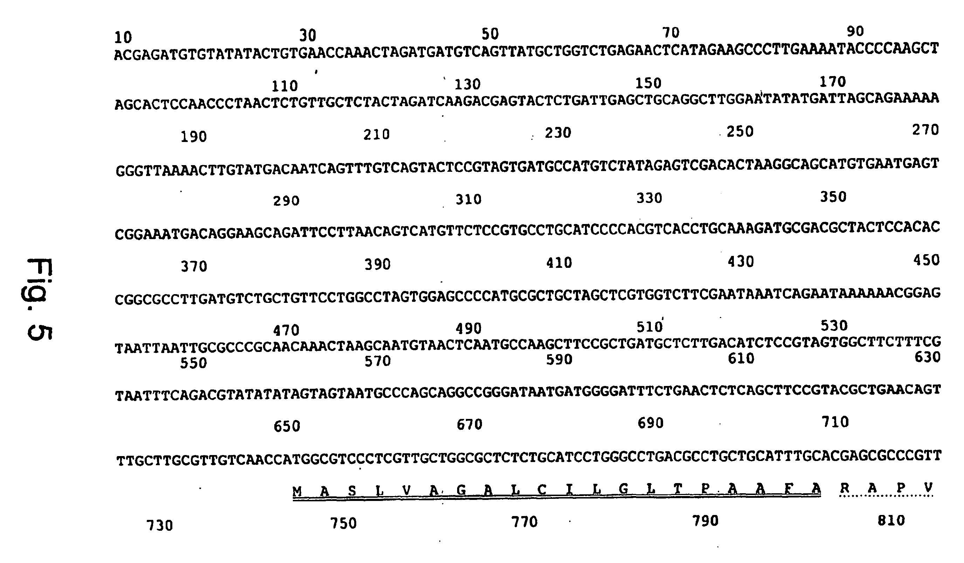

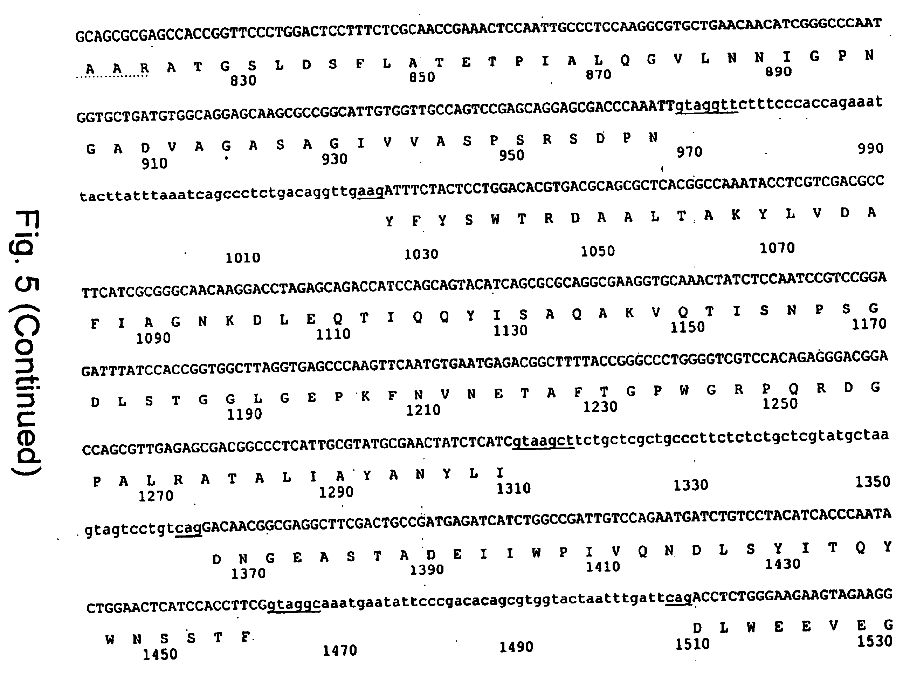

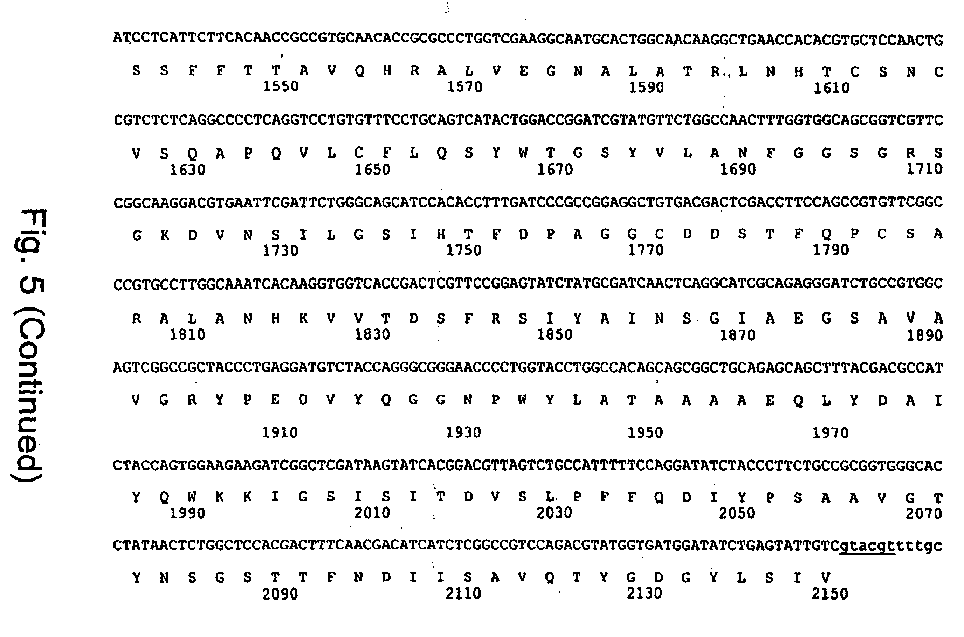

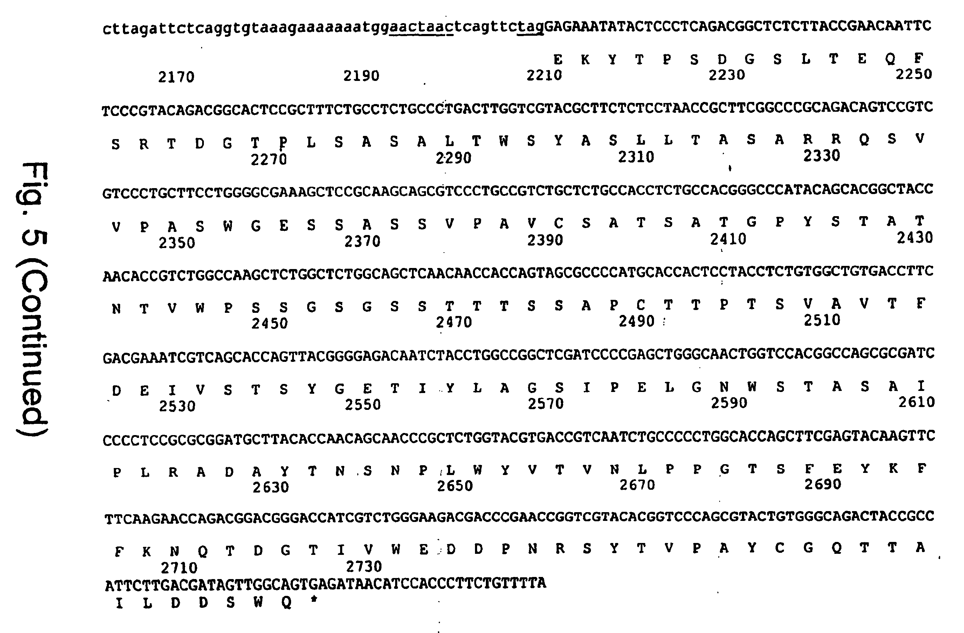

*A. niger* G1 glucoamylase gene is shown in SEQ ID NO: 8 *T. emersonii* glucoamylase gene with introns is shown in fig. 5 and SEQ ID NO: 33. The introns are shown in Fig. 5.

### Plasmids:

pJSO026 (*S. cerevisiae* expression plasmid) (J.S. Okkels, (1996)"A URA3-promoter deletion in a pYES vector increases the expression level of a fungal lipase in Saccharomyces cerevisiae. Recombinant DNA Biotechnology III: The Integration of Biological and Engineering Sciences, vol. 782 of the Annals of the New York Academy of Sciences) More specifically, the expression plasmid pJSO26 is derived from pYES 2.0 by replacing the inducible GAL1-promoter of pYES 2.0 with the constitutively expressed TPI (triose phosphate isomerase)-promoter from *Saccharomyces cerevisiae* (Albert and Karwasaki, (1982), J. Mol. Appl Genet., 1, 419-434), and deleting a part of the URA3 promoter.
pJaL497; Construction of this plasmid is described in Example 5
pJaL507; Construction of this plasmid is described in Example 5
pJaL510; Construction of this plasmid is described in Example 5
pJaL511; Construction of this plasmid is described in Example 5
pJaL518; Construction of this plasmid is described in Example 6
pCaHj483; Construction of this plasmid is described in Example 6
pJRoy10; Construction of this plasmid is described in Example 6
pJRoy17; Construction of this plasmid is described in Example 6
pSM0127; Construction of this plasmid is described in Example 6
pCR™II; Available from Invitrogen Corporation, San Diego, CA, USA.

### Equipment:

Automatic DNA Sequencer (Applied Biosystems Model 377)

| Media: | |
|---|---|
| SC-ura medium: | |
| Yeast Nitrogen w/o ami | 7.5 g |
| Bernsteinsaüre (Ravsyre) | 11.3 g |
| NaOH | 6.8 g |
| Casaminoacid w/o vit | 5.6 g |
| Tryptophan | 0.1 g |
| Dest. water ad | 1000 ml |

Autoclaved for 20 minutes at 121°C.

From a sterile stock solution of 5% Threonin 4 ml is added to a volume of 900 ml together with 100 ml of a sterile 20% glucose.

| YPD medium: | |
|---|---|
| Yeast extract | 10 g |
| Peptone | 20 g |
| Dest. water ad | 1000 ml |

Autoclaved for 20 minutes at 121°C
100 ml of a sterile 20% glucose is added to 900 ml.

### Methods:

### Determination of AGU activity

One Novo Amyloglucosidase Unit (AGU) is defined as the amount of enzyme which hydrolyzes 1 micromole maltose per minute under the following standard conditions:
- Substrate: maltose
- Temperature: 25°C
- pH: 4.3 (acetate buffer)
- Reaction time: 30 minutes

A detailed description of the analytical method (AF22) is available on request.

### Determination of PUN activity

PUN is defined as the amount of enzyme which hydrolyzes pullulan (0.2 % pullulan, 40°C, pH 5.0), liberating reducing carbohydrate with a reducing power equivalent to 1 micro-mol glucose pr. minute.

### Determination of AFAU activity

The activity is determined in AFAU calculated as the reduction in starch concentration at pH 2.5, 40°C, 0.17 g/l starch and determined by an iodine-starch reaction.

### Thermal Stability I (T½ (half-life) Determination of AMG

The thermal stability of glucoamylase (determined as T½ (half-life)) is tested using the following method: 950 microliter 50 mM sodium acetate buffer (pH 4.5) (NaOAc) is incubated for 5 minutes at 70°C. 50 microliter enzyme in buffer (4 AGU/ml) is added. 2 x 40 microliter samples are taken at fixed periods between 0 and 360 minutes and chilled on ice. After chilling the samples the residual enzyme activity is measured using the AGU determination assay (described above).

The activity (AGU/ml) measured before incubation (0 minutes) is used as reference (100%). T_{1/2} is the period of time until which the percent relative activity is decreased to 50%.

### Determination of thermal stability II

1600 microliter of a supernatant and 400 microliter of 0.5M NaAC pH 4.5 is mixed.
7 eppendorph tubes each containing 250 microliter of the mixture are incubated in a Perkin Elmer thermocycler at 68°C or 70°C for 0, 5, 10, 20, 30, 45 and 60 minutes.

100 microliter from each mixture is mixed with 100 microliter of 5 mM CNPG3 (2-chloro-4-Nitrophenyl-Alpha-Maltotrioside from genzyme) in microtiterwells. After incubation for 30 minutes at 37°C the absorbance is measured at 405 nm.

### Determination of Specific activity of a glucoamylase

750 microL substrate is incubated 5 minutes at selected temperatures, such as 37°C, 60°C or 70°C.

50 microL enzyme diluted in sodium acetate is added,and the activity was determined using the AGU standard method described above. The kinetic parameters: Kcat and Km are measured at 45°C by adding 50 microL enzyme diluted in sodium acetate to preheated 750 microL substrate. Aliquots of 100 microL are removed after 0, 3, 6, 9 and 12 minutes and transferred to 100 microL 0.4M Sodium hydroxide to stop the reaction. A blank is included.

20 microL is transferred to a Micro titre plates and 200 microL GOD-Perid solution is added. Absorbance is measured at 650 nm after 30 minutes incubation at room temperature. Glucose is used as standard, and the specific activity is calculated as k_{cat} (sec. ⁻¹)

### Transformation of Aspergillus oryzae (general procedure)

100 ml of YPD (Sherman et al., (1981), Methods in Yeast Genetics, Cold Spring Harbor Laboratory) is inoculated with spores of *A. oryzae* and incubated with shaking for about 24 hours. The mycelium is harvested by filtration through miracloth and washed with 200 ml of 0.6 M MgSO₄. The mycelium is suspended in 15 ml of 1.2 M MgSO₄, 10 mM NaH₂PO₄, pH 5.8. The suspension is cooled on ice and 1 ml of buffer containing 120 mg of Novozym™ 234 is added. After 5 min., 1 ml of 12 mg/ml BSA (Sigma type H25) is added and incubation with gentle agitation continued for 1.5-2.5 hours at 37C until a large number of protoplasts is visible in a sample inspected under the microscope.

The suspension is filtered through miracloth, the filtrate transferred to a sterile tube and overlayed with 5 ml of 0.6 M sorbitol, 100 mM Tris-HCl, pH 7.0. Centrifugation is performed for 15 min. at 1000 g and the protoplasts are collected from the top of the MgSO₄ cushion. 2 volumes of STC (1.2 M sorbitol, 10 mM Tris-HCl, pH 7.5, 10 mM CaCl₂) are added to the protoplast suspension and the mixture is centrifugated for 5 min. at 1000 g. The protoplast pellet is resuspended in 3 ml of STC and repelleted. This is repeated. Finally, the protoplasts are resuspended in 0.2-1 ml of STC.

100 µl of protoplast suspension are mixed with 5-25 µg of p3SR2 (an *A. nidulans* amdS gene carrying plasmid described in Hynes et al., Mol. and Cel. Biol., Vol. 3, No. 8, 1430-1439, Aug, 1983) in 10 µl of STC. The mixture is left at room temperature for 25 min. 0.2 ml of 60% PEG 4000 (BDH 29576), 10 mM CaCl₂ and 10 mM Tris-HCl, pH 7.5 is added and carefully mixed (twice) and finally 0.85 ml of the same solution are added and carefully mixed. The mixture is left at room temperature for 25 min., spun at 2.500 g for 15 min. and the pellet is resuspended in 2 ml of 1.2M sorbitol. After one more sedimentation the protoplasts are spread on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth. After incubation for 4-7 days at 37C spores are picked, suspended in sterile water and spread for single colonies. This procedure is repeated and spores of a single colony after the second re-isolation are stored as a defined transformant.

### Fed batch fermentation

Fed batch fermentation is performed in a medium comprising maltodextrin as a carbon source, urea as a nitrogen source and yeast extract. The fed batch fermentation is performed by inoculating a shake flask culture of fungal host cells in question into a medium comprising 3.5% of the carbon source and 0.5% of the nitrogen source. After 24 hours of cultivation at pH 5.0 and 34°C the continuous supply of additional carbon and nitrogen sources are initiated. The carbon source is kept as the limiting factor and it is secured that oxygen is present in excess. The fed batch cultivation is continued for 4 days, after which the enzymes can be recovered by centrifugation, ultrafiltration, clear filtration and germ filtration. Further purification may be done by anionexchange chromatographic methods known in the art.

### Transformation of Saccharomyces cerevisiae YNG318

The DNA fragments and the opened vectors are mixed and transformed into the yeast *Saccharomyces cerevisiae* YNG318 by standard methods.

### EXAMPLES

### Example 1

### Purification

3500 ml *T. emersonii* culture broth from wild-type fermentation with 0.05 AGU/ml was centrifuged at 9000 rpm followed by vacuum filtration through filter paper and finally a blank filtration. The following procedure was then used to purify the enzyme:
Phenyl Sepharose (250 ml): 1,3 M AMS/10 mM Tris/2 mM CaCl₂, pH 7; elution with 10 mM Tris/2 mM CaCl₂, pH 7.
Dialysis: 20 mM NaAc, 2mM CaCl₂, pH 5.
Q Sepharose (100 ml) : 20 mM NaAc, 2mM CaCl₂, pH 5; elution with a linear gradient from 0-0.4 M NaCl over 10 column volumes. Dialysis: 20 mM NaAc, 2 mM CaCl₂, pH 5.
Colour removal: 0.5% coal in 10 minutes.
Q Sepharose (20 ml): 20 mM NaAc, 2mM CaCl₂, pH 4.5; elution with a linear gradient from 0-0.4 M NaCl over 10 column volumes.
Dialysis: 20 mM NaAc, 2mM CaCl₂, pH 5.
S Sepharose (1 ml): 5 mM citric acid, pH 2.9; elution with a linear gradient from 0-0.3 M NaCl over 10 column volume.

A purity of the enzyme of more than 90% was obtained after the S Sepharose step.

### Example 2

### Characterisation of the Talaromyces emersonii glucoamylase

The purified *Talaromyces emersonii* CBS 793.97 glucoamylase was used for characterisation.

### Molecular weight (Mᵥ)

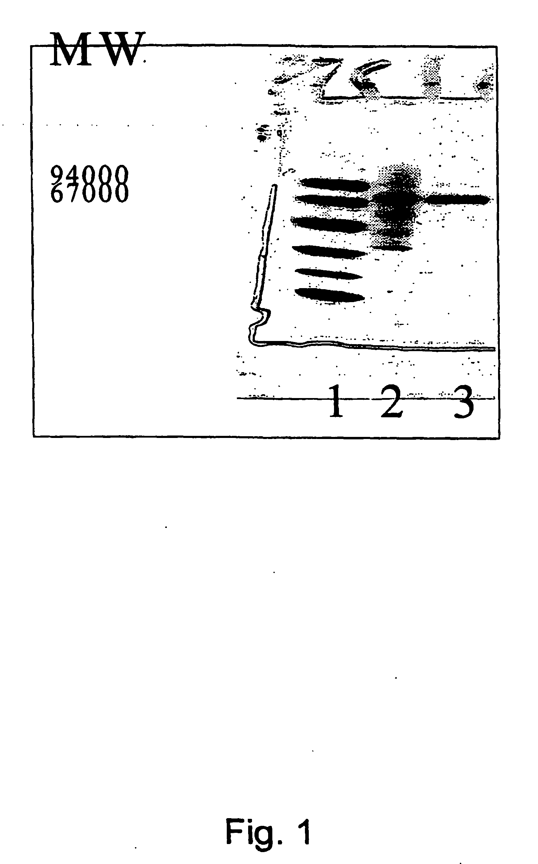

The molecular weight was determined by SDS-PAGE to around 70 kDa as shown in Figure 1.

### pI

The pI was determined to lie below 3.5 by isoelectrical focusing (Amploline PAG, pH 3.5-9.5 from Pharmacia).

### pH profile

The pH-activity dependency of the *Talaromyces emersonii* glucoamylase was determined and compared with profile of *Aspergillus* niger glucoamylase.

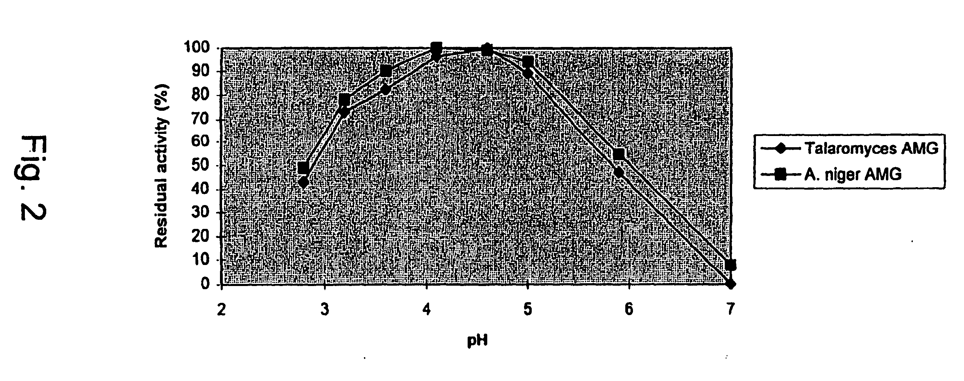

The pH activity profile was determined using 0.5% maltose as substrate in 0.1 M sodium acetate at 60°C. The pH was measured in duple samples comprising 0.1-1 AGU/ml. The result of the test is shown in Figure 2.

### Temperature profile

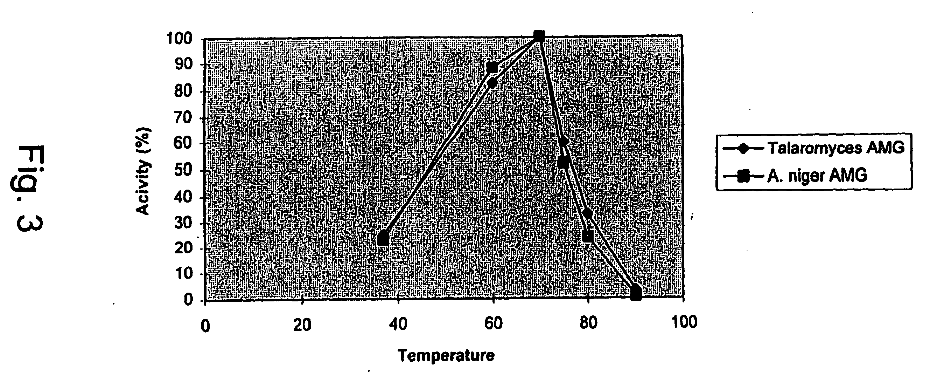

The temperature-activity dependency of the *Talaromyces emersonii* glucoamylase of the invention was determined and compared with the profile of *Aspergillus niger* glucoamylase. 200 µl 0.5% maltose, pH 4.3 was incubated at 37, 50, 60, 70, 75, 80 and 90°C and the reaction was started by adding 10 µl enzyme (0.25 AGU/ml); reaction time was 10 minutes. The result of the test is shown in Figure 3.

### Temperature stability - T½ (half-life)

The thermal stability of the *Talaromyces emersonii* glucoamylase was determined and compared with the thermal stability of *Aspergillus niger* glucoamylase.
The method used is described above in the "Material and Methods" section as "Thermal Stability I (T½ (half-life) determination of AMG".

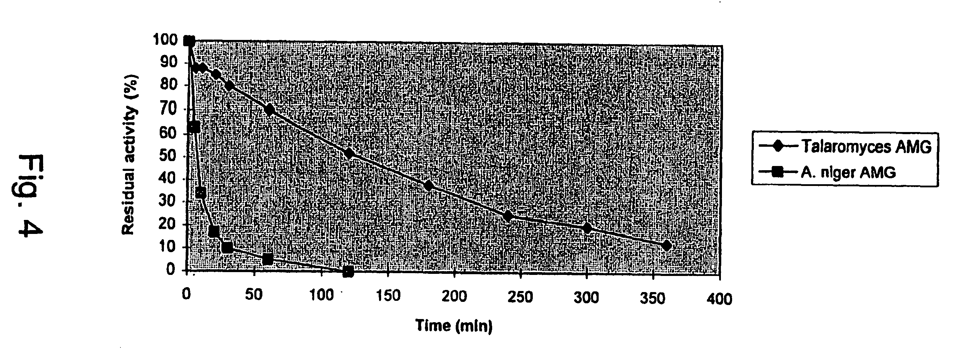

The T½ of the *Talaromyces emersonii* glucoamylase was determined to about 120 minutes at 70°C. The T½ of the *Aspergillus niger* glucoamylase was determined to 7 minutes under the same conditions (See Figure 4).

### Specific activity

The extension coefficient was determined to: ε = 2.44 ml/mg*cm on basis of absorbency at 280 nm and protein concentration. The specific activity towards maltose at 37°C was then calculated to 7.3 AGU/mg. Purity of the sample was approximately 90% and a corrected specific activity is therefore 8.0 AGU/mg. Following specific activities were measured:

| AMG | Specific activity (AGU/mg) | | |
|---|---|---|---|
| | 37°C | 60°C | 70°C |
| *T. emersonii* * | 8.0 | 21 | 27 |
| *A. niger* | 2.0 | 6.6 | 8.0 |

| | | | |
|---|---|---|---|
| *) Estimated for pure enzyme. | | | |

### EXAMPLE 3

### Sequencing of the N-terminal of T. emersonii glucoamylase

The N-terminal amino acid sequence of *T. emersonii* glucoamylase was determined following SDS-PAGE and electroblotting onto a PVDF-membrane. Peptides were derived from reduced and S-carboxymethylated glucoamylase by cleaving with a lysyl-specific protease. The resulting peptides were fractionated and re-purified using RP-HPLC before subjected to N-terminal sequence determination.
N-terminal sequence (SEQ ID NO: 1) :
Peptide 1 (SED ID NO: 2) :
Peptide 2 (SEQ ID NO : 3) :
Peptide 3 (SEQ ID NO: 4) :
Peptide 4 (SEQ ID NO: 5)
   Thr Xaa Ala Ala Ala Glu Gln Leu Tyr Asp Ala Ile Tyr Gln Trp Lys
Peptide 5 (SEQ ID NO : 6) : Xaa denoted a residue that could not be assigned.

### EXAMPLE 4

### The full length T. emersonii_glucoamylase

The full length T. emersonii glucoamylase amino acid sequence shown in SEQ ID NO: 7 was identified using standard methods.

### Example 5

### Cloning and sequencing of the Talaromyces emersonii glucoamylase gene

### PCR cloning parts of the Talaromyces emersonii AMG gene

For cloning of the *Talaromyces emersonii* AMG gene degenerated primers shown in table 1 was designed for PCR amplification of part of the AMG gene.

**Table 1**

| Primer no: | sequence | comments |
|---|---|---|
| | | N-terminal |
| 102434(SEQ ID NO:10) | | |
| 102435 (SEQ ID NO:11) | | 5' primers |
| | | |
| | | Active site |
| 117360 (SEQ ID NO:12) | | consensus 3' |
| 117361(SEQ ID NO:13) | | primers |
| | | |
| | | C-Terminal |
| 127420 (SEQ ID NO:14) | | 3' primers |
| | | |

Genomic DNA *from Talaromyces emersonii* was prepared from protoplasts made by standard procedures [cf.e,.g., Christensen et al. Biotechnology 1989 6 1419-1422] and was used as template in the PCR reaction. Amplification reaction were performed in 100 µl volumes containing 2.5 units Taq-polymerase, 100 ng of *A.oryzae* genomic DNA, 50 mM KCl, 10 mM Tris-HCl pH 8.0,1.5 mM MgCl₂, 250 nM of each dNTP, and 100pM of each of the following primers sets: 102434/117360, 102434/117361, 102435/117360, 102434/117361, 102434/127420, and 102434/127420.

Amplification was carried out in a Perkin-Elmer Cetus DNA Termal 480, and consisted of one cycle of 3 minutes at 94°C, followed by 30 cycles of 1 minutes at 94°C, 30 seconds at 40°C, and 1 minutes at 72°C. Only the PCR reaction 102434/117360 gave products. Four bands was detected with the following sizes 1400, 800, 650, and 525bp. All four bands were purified and cloned into the vector pCR^{®}2.1 (Invitrogen^{®}). Sequencing of a few clones from each band and sequence comparisons to the *A.niger* AMG, releaved that a clone from the 650 bp band encodes for the N-terminal part of the Talaromyces emersonii AMG. This clone was designated pJaL497.

To obtained more of the gene a specific primer (123036: 5'-GTGAGCCCAAGTTCAATGTG- 3' (SEQ ID NO :15) was made out from the sequence of clone pJaL497. The primer set 123036/127420 was used for PCR on Talaromyces genomic DNA and a single fragment on 1500 bp was obtained. The PCR fragment was cloned into the vector pCR^{®}2.1 and sequenced. By sequencing the clone was confirmed to encoded the C-terminal part of the *Talaromyces emersonii* AMG. The clone was designated pJaL507.

### Genomic restriction mapping and cloning of a genomic clone (s)

Taken together the two clones pJaL497 and pJaL507 covered about 95% of the AMG gene. In order to clone the missing part of the AMG gene a genomic restriction map was constructed by using the two PCR fragment as probes to a Southern blot of Talaromyces emersonii genomic DNA digested with single or a combination of a number of restriction enzymes. This shows that the Talaromyces emersonii AMG gene is located on two EcoRI fragment on about 5.6 kb and 6.3 kb, respectively.

*Talaromyces emersonii* genomic DNA was digested with EcoRI and fragments with the size between 4-7 kb was purified and used for construction of a partially genome library in Lambda ZAP II as described by the manufactory instruction(stratagene). The library was first screened using the 0.7 kb EcoRI fragment from pJaL497 (encoding the N-terminal half of the AMG gene) as probe to get the start of the AMG gene. One clone was obtained and designated pJaL511. In a second screening of the library using a 0.75 kb EcoRV fragment from pJaL507 (encoding the C-terminal half of the AMG gene) as probe in order to get the C-terminal end of the AMG gene. One clone was obtained and designated pJaL510.

### Sequence analysis of the Talaromyces emersonii AMG gene

The AMG gene sequence was obtained by sequencing on the plasmids: pJaL497, pJaL507, pJaL510, and pJaL511 and on subclones hereof with the standard reverse and forward primers for pUC. Remaining gabs were closed by using specific oligonucleotide as primers.

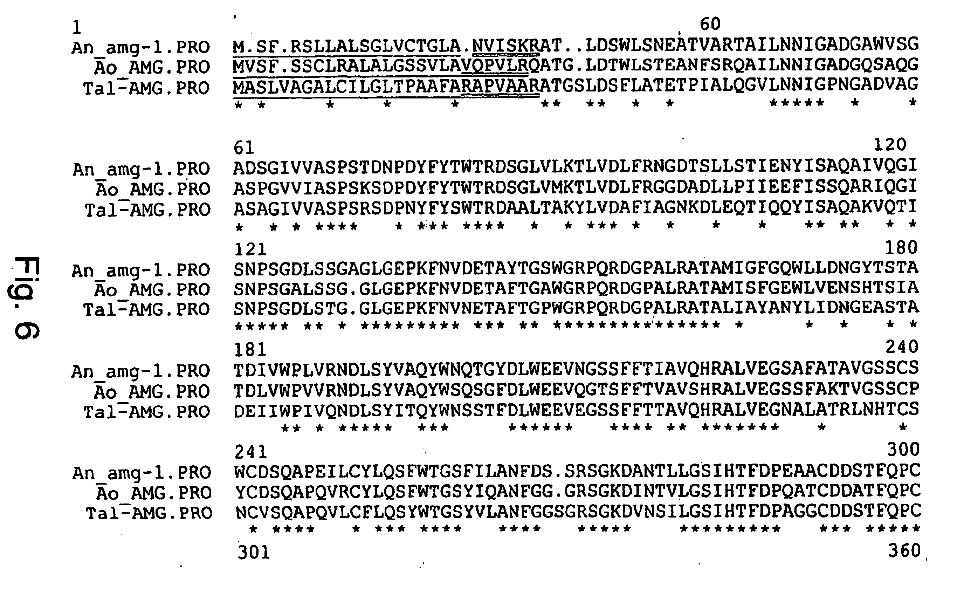

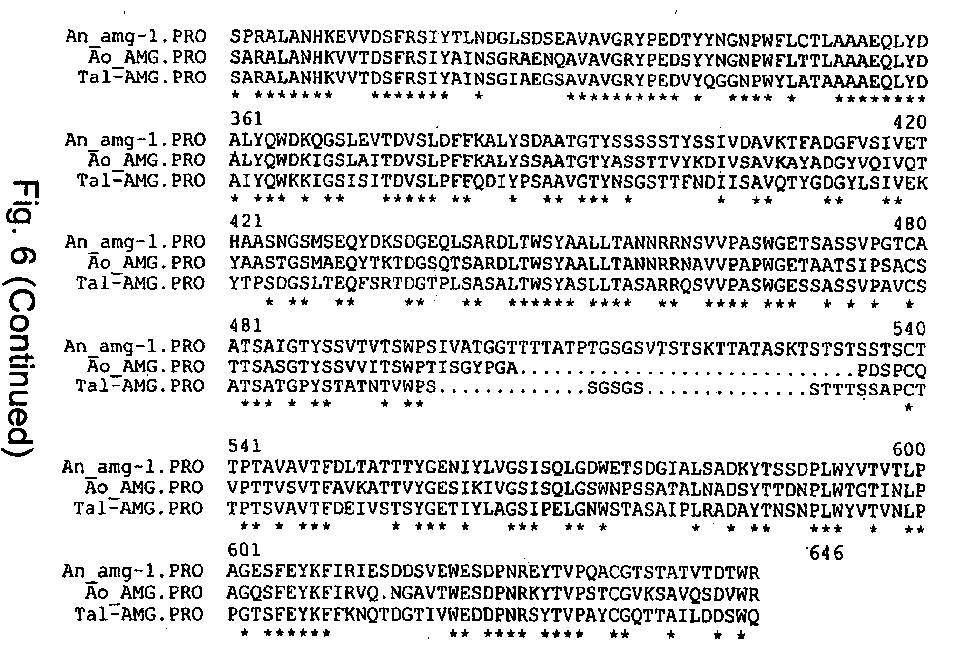

Potential introns were found by comparing the sequence with consensus sequences for introns in *Aspergillus* and with the A.niger AMG sequence. The *Talaromyces emersonii* AMG nucleotide sequence has an open reading frame encoding a protein on 618 amino acid, interrupted by four introns of 57 bp, 55 bp, 48 bp, and 59 bp, respectively. The nucleotide sequence (with introns) and deduced amino acid sequence is shown in Fig. 5. The DNA sequence (with introns) is also shown in SEQ ID NO: 33 and the *Talaromyces emersonii* AMG sequence (with signal sequence from 1 to 27) is shown in SEQ ID NO: 34. Comparison of the deduced amino acid sequence with the *A*.*oryzae* AMG and *A.niger* AMG shows an identity of 60.1 % and 60.5 %, respectively. Alignment of the amino acid sequences shown in Fig. 6 shows that the *Talaromyces* AMG has a very short hinge between the catalytic domain and the starch binding domain, which is also seen for the *A.oryzae* AMG.

### Example 6

### Construction of the Aspergillus vector pCaHj483

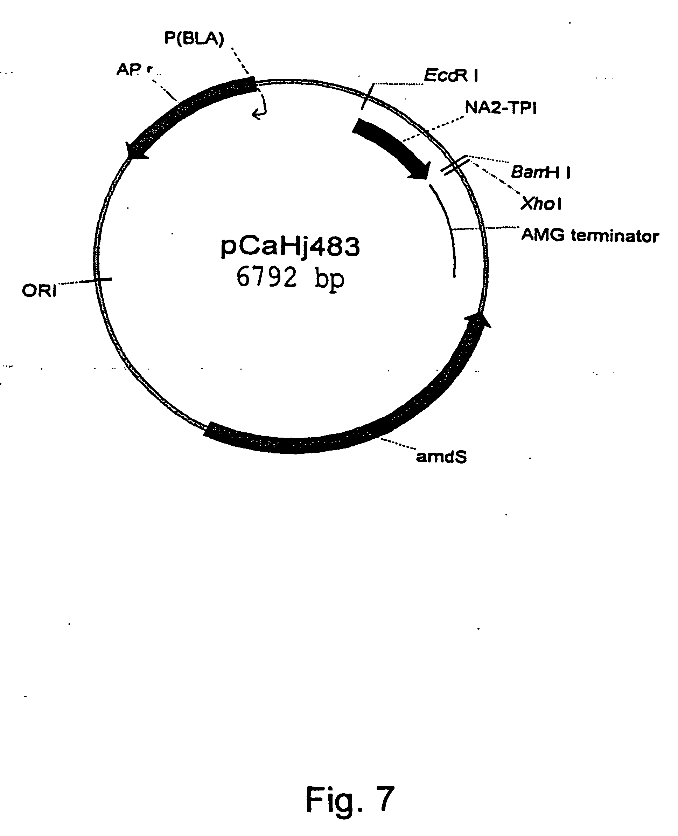

Construction of pCaHj483 is depicted in Fig. 7. Said plasmid is build from the following fragments:
a) The vector pToC65 (WO 91/17243) cut with *EcoRI* and *XbaI.*
b) A 2.7 kb *XbaI* fragment from *A. nidulans* carrying the amds gene (C. M. Corrick et al., Gene 53, (1987), 63-71). The amdS gene is used as a selective marker in fungal transformations. The *amdS* gene has been modified so that the *BamHI* site normally present in the gene is destroyed. This has been done by introducing a silent point mutation using the primer: 5'-AGAAATCGGGTATCCTTTCAG- 3' (SEQ ID NO:16)
c) A 0.6 kb *EcoRI*/*HamHI* fragment carrying the A. *niger* NA2 promoter fused to a 60bp DNA fragment of the sequence encoding the 5' untranslated end of the mRNA of the *A. nidulans* tpi gene. The NA2 promoter was isolated from the plasmid pNA2 (described in WO 89/01969) and fused to the 60 bp tpi sequence by PCR. The primer encoding the 60 bp tpi sequence had the following sequence:
d) A 675 bp *XbaI* fragment carrying the A. niger glucoamylase transcription terminator. The fragment was isolated from the plasmid pICAMG/Term (described in EP 0238 023).

The *BamHI* site of fragment c was connected to the *XbaI* site in front of the transcription terminator on fragment d via the pIC19R linker (*BamHI* to *XbaI*)

### Construction of a AMG expression plasmid, pJaL518

The coding region of the *Talaromyces emersonii* AMG gene was amplified by PCR, using the following two oligonucleotides primers: 139746:
5'-GACAGATCTCCACCATGGCCCTCGTTG 3' (SEQ ID NO:18); and
primer 139747:
5'-GACCTCGAGTCATGCCAACTACTGCC 3' (SEQ ID NO:19). The underlined regions indicate sequences present in the *Talaromyces emersonii* AMG gene. To facilitate cloning a restriction enzyme site was inserted into the 5' end of each primer; primer 139746 contains a BglII site and primer 139747 contains a XhoI site.
Talaromyces emersonii genomic DNA was used as template in the PCR reaction. The reaction was performed in a volume of 100 µl containing 2.5 units Taq polymerase, 100 ng of pSO2, 250 nM of each dNTP, and 10 pmol of each of the two primers described above in a reaction buffer of 50 mM KCl, 10 mM Tris-HCl pH 8.0, 1.5 mM MgCl₂.

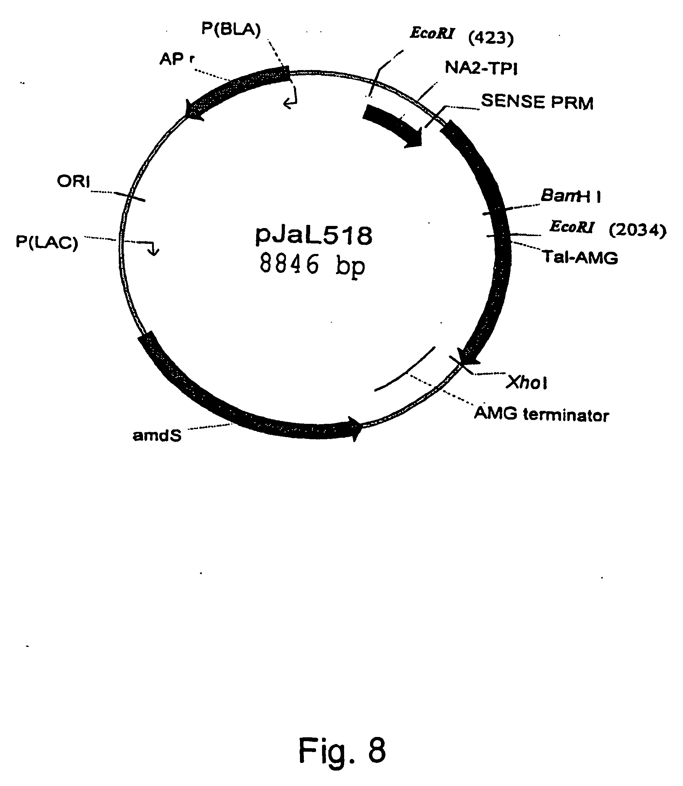

Amplification was carried out in a Perkin-Elmer Cetus DNA Termal 480, and consisted of one cycle of 3 minutes at 94°C, followed by 25 cycles of 1 minute at 94°C, 30 seconds at 55°C, and 1 minute at 72°C. The PCR reaction produced a single DNA fragment of 2099 bp in length. This fragment was digested with BglII and XhoI and isolated by gel electrophoresis, purified, and cloned into pCaHj483 digested with BamHI and XhoI, resulting in a plasmid which was designated pJaL518. Thus, the construction of the plasmid pJal518 resulted in a fungal expression plasmid for the *Talaromyces emersonii* AMG gene (Fig. 8).

### Construction of the Aspergillus niger strain. SMO110

### 1. Cloning of A.niger pyrG gene

A library of *A.niger* BO-1 was created in EMBL4 as described by the manufactory instructions. The library was screened with a DIG labelled oligonucleotides (PyrG: 5'-CCCTCACCAGGGGAATGCTGCAGTTGATG- 3' (SEQ ID NO:20) which was designed from the published *Aspergillus niger* sequence (Wilson et al. Nucleic Acids Res. 16, (1988), 2339-2339). A positive EMBL4 clone which hybridized to the DIG probe was isolated from the BO-1 library, and a 3.9 kb Xba1 fragment containing the pyrG gene was subcloned from the EMBL4 clone and clone into pUC118 to create pJRoy10.

### 2. Cloning of the A.niger glucoamylase (AMG) gene

The above *A.niger* BO-1 library was screened with a DIG labelled PCR fragment generated by amplification on *A.niger* genomic DNA with the following oligonucleotides, 950847:
5'-CGCCATTCTCGGCGACTT-3' (SEQ ID NO:21), and oligonucleotide 951216:
5'-CGCCGCGGTATTCTGCAG-3' (SEQ ID NO:22), which was designed from the published *Aspergillus niger* sequence (Boel et al., EMBO J. 3, (1984), 1581-1585). A positive EMBL4 clone which hybridized to the DIG probe was isolated from the BO-1 library, and a 4.0 kb SpeI fragment containing the AMG gene was subcloned from the EMBL4 clone and clone into pBluescriptSK+ generating plasmid pJRoy17a.

### 3. Construction of the A. niger AMG Disruption Cassette

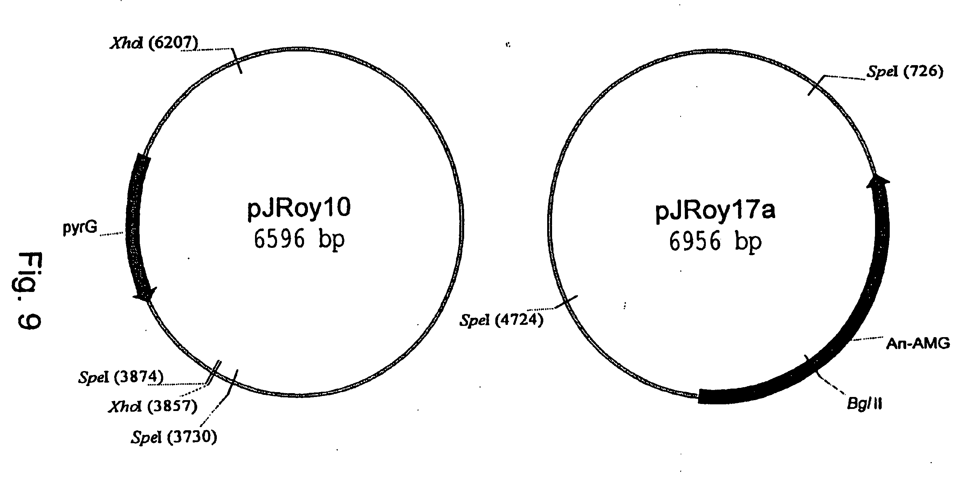

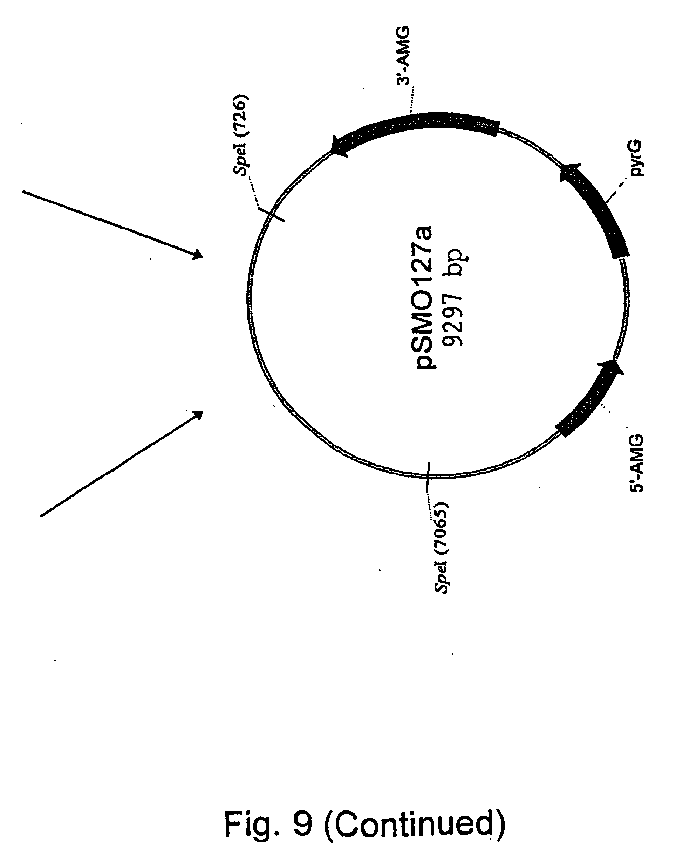

A 2.3 kb SpeI-XhoI fragment containing *pyrG* was gel isolated from pJRoy10 and the restricted ends filled in with Klenow polymerase. The fragment was inserted into the BglII site of pJRoy17 which cuts within the AMG gene creating plasmid pSMO127 (Fig. 9). Between the two SpeI sites of pSMO127a is contained the 2.3 kb *pyrG* gene flanked by 2.2 kb and 2.3 kb 5' and 3' AMG, respectively.

### 4. Construction of a A. niger strain disrupted for AMG, SMO110

*A.niger* JRoyP3 is a spontaneously pyrG mutant of *A.niger* BO-1, which was selected for the growth on a plate containing 5'-fluoro-orotic acid (5'-FOA). The pyrG gene encodes orotidine 5'-phosphate carboxylase and its deficient mutant can be characterized as uridine auxotroph. The identity of pyrG mutant was confirmed by the complementation of the growth on a minimal medium with *A.nidulans* pyrG gene.

Twenty micrograms of the plasmid pSMO127 was digested with SpeI. The DNA was resolved on an 0.8% agarose gel and the 6 kb consisting of the linear disruption cassette was gel isolated. The linear DNA was transformed into strain JRoyP3. Genomic DNA was prepared from 200 transformants which was then digested with SpeI. The gel-resolved DNA was transferred to a hybond nylon filter, and hybridized to a non-radioactive DIG probe consisting of the AMG open reading frame. A gene replacement of the disruption cassette into the AMG locus would result in an increase of the wild type 4 kb AMG band to 6.3 kb, an increase due to the 2.3 kb *pyrG* gene. One transformant #110 with the above characteristics was selected for further analysis.

The transformant #110 were grown in 25 ml shake flasks containing YPM media. Strains BO-1 and parent strain JRoyP3 were grown as AMG producing controls. After 3 days, 30µl of clear supernatants were run on a 8-16% SDS PAGE Novex gel. No AMG band was seen in transformant #110, while large bands of AMG were produced in the positive control strain BO-1 and parent strain JRoyP3. Transformant #110 was named SMO110.

### Expression of Talaromyces emersonii AMG in Aspergillus ozyzae and Aspergillus niger

The strains JaL228 and SM0110 was transformed with pJaL518 as described by Christensen et al.; Biotechnology 1988 6 1419-1422. Typically, *A. oryzae* mycelia was grown in a rich nutrient broth. The mycelia were separated from the broth by filtration. The enzyme preparation Novozyme^{®} (Novo Nordisk) was added to the mycelia in osmotically stabilizing buffer such as 1.2 M MgSO₄ buffered to pH 5.0 with sodium phosphate. The suspension was incubated for 60 minutes at 37°C with agitation. The protoplast was filtered through mira-cloth to remove mycelial debris. The protoplast was harvested and washed twice with STC (1.2 M sorbitol, 10 mM CaCl₂, 10 mM Tris-HCl pH 7.5). The protoplast was finally resuspended in 200-1000 µl STC.

For transformation 5 µg DNA was added to -100 µl protoplast suspension and then 200 µl PEG solution (60% PEG 4000, 10 mM CaCl₂, 10 mM Tris-HCl pH 7.5) was added and the mixture was incubated for 20 minutes at room temperature. The protoplast were harvested and washed twice with 1.2 M sorbitol. The protoplast was finally resuspended 200 µl 1.2 M sorbitol, plated on selective plates (minimal medium + 10 g/l Bacto-Agar (Difco), and incubated at 37°C. After 3-4 days of growth at 37°C, stable transformants appear as vigorously growing and sporulating colonies. Transformants was spore isolated twice.

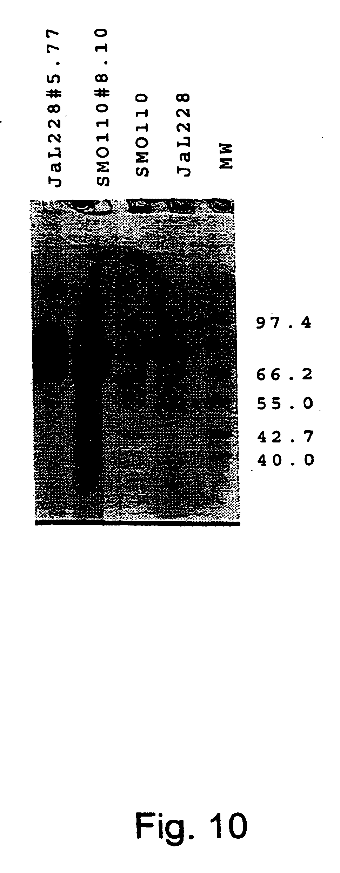

Transformants was grown in shake flask for 4 days at 30°C in 100 ml YPM medium (2 g/l yeast extract, 2 g/l peptone, and 2% maltose). Supernatants were tested for AMG activity as described and analyzed on SDS page gel (Fig. 10).

### EXAMPLE 7

### Removal_ of the four introns from the Talaromyces emersonii AMG DNA sequence for expression in yeast.

For each exon a PCR reaction was made with primers containing overlap to the next exon. Tal 1 and Tal 4 contain an overlap with the yeast vector pJS0026.
Exon 1: Tal 1 was used as the 5' primer and Tal 5 as the 3' primer and the genomic sequence coding for AMG was used as the template. Exon 2: Tal 6 was used as the 5' primer and Tal 7 was used as the 3' primer and the genomic sequence coding for AMG was used as the template. Exon 3: Tal 8 was used as the 5' primer and Tal 9 was used as the 3' primer and the genomic sequence coding for AMG was used as the template. Exon 4: Tal 10 was used as the 5' primer and Tal 11 was used as the 3' primer and the genomic sequence coding for AMG was used as the template. Exon 5: Tal 12 was used as the 5' primer and Tal 4 was used as the 3' primer and the genomic sequence coding for AMG was used as the template.

A final PCR reaction was performed to combine the 5 exons to a sequence containing the complete coding sequence. In this PCR reaction the 5 fragments from the first PCR reactions were used as template and Tal 1 was used as the 5' primer and Tal4 was used as the 3' primer.

This final PCR fragment containing the coding region was used in an in vivo recombination in yeast together with pJSO026 cut with the restriction enzymes SmaI(or BamHI) and XbaI (to remove the coding region and at the same time create an overlap of about 20 bp in each end to make a recombination event possible).
Tal 1: 5'-CAA TAT AAA CGA CGG TAC CCG GGA GAT CTC CAC CATG GCG TCC CTC GTT G-3' (SEQ ID NO:23);
Tal 4: 5'-CTA ATT ACA TCA TGC GGC CCT CTA GAT CAC TGC CAA CTA TCG TC-3' (SEQ ID NO:24);
Tal 5: 5'-AAT TTG GGT CGC TCC TGC TCG-3' (SEQ ID NO:25);
Tal 6: 5'-CGA GCA GGA GCG ACC CAA ATT ATT TCT ACT CCT GGA CAC G-3' (SEQ ID NO: 26);
Tal 7: 5'-GAT GAG ATA GTT CGC ATA CG-3' (SEQ ID NO: 27);
Tal 8: 5'-CGT ATG CGA ACT ATC TCA TCG ACA ACG GCG AGG CTT CGA CTG C-3' (SEQ ID NO:28) ;
Tal 9: 5'-CGA AGG TGG ATG AGT TCC AG-3' (SEQ ID NO: 29);
Tal 10: 5'-CTG GAA CTC ATC CAC CTT CGA CCT CTG GGA AGA AGT AGA AGG-3' (SEQ ID NO: 30)
Tal 11: 5'-GAC AAT ACT CAG ATA TCC ATC-3' (SEQ ID NO: 31)
Tal 12 : 5'-GAT GGA TAT CTG AGT ATT GTC GAG AAA TAT ACT CCC TCA GAC G-3' (SEQ ID NO: 32)

### EXAMPLE 8

### Expression of Talaromyces emersonii glucoamylase in yeast

To express *Talaromyces emersonii* AMG in the yeast *Saccharomyces cerevisiae* YNG318 the yeast expression vector pJSO26 was constructed as described in the "Material and Methods" section above.

PJSO26 comprising the DNA sequence encoding the *Talaromyces* AMG was transformed into the yeast by standard methods (cf. Sambrooks et al., (1989), Molecular Cloning: A Laboratory Manual, 2nd Ed., Cold Spring Harbor)

The yeast cells were grown at 30°C for 3 days in Sc-ura medium followed by growth for 3 days in YPD. The culture was then centrifuged and the supernatant was used for the thermostability assay described in the "Materials and Method" section.

### Thermal Stability of the Talaromyces AMG expressed in yeast at 68°C.

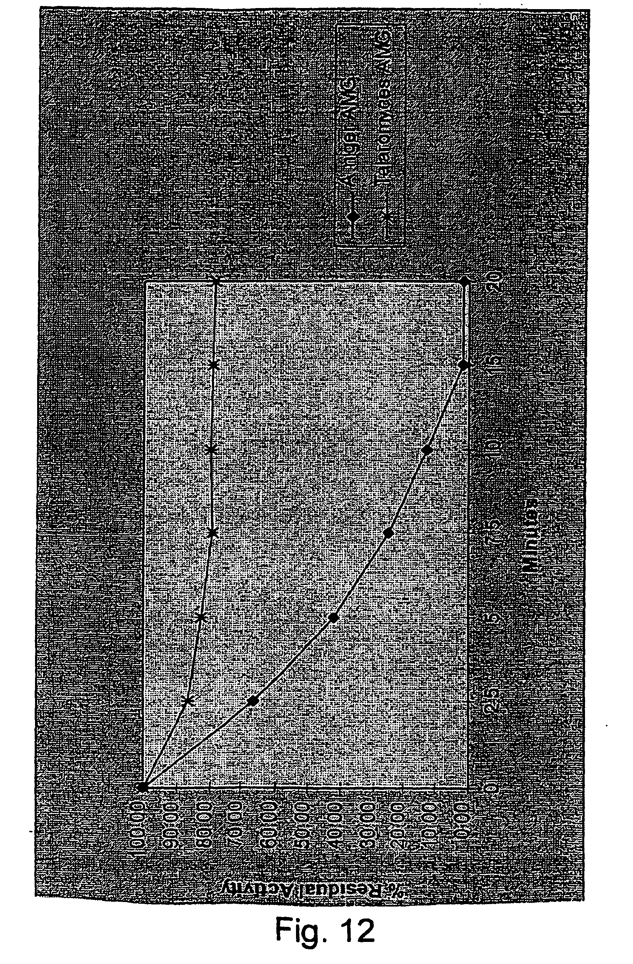

The fermentation broth of the *Talaromyces emersonii* AMG expressed in yeast (*Saccharomyces cerevisiae* YNG318) was used for determination of the thermal stability at 68°C using the method described above under "Determination of thermal stability II". The result of the test is shown in Figure 12.

### EXAMPLE 9

### Purification of recombinant Talaromyces AMC produced using A. niger HowB112

200 ml culture broth from fermentation of *A. niger* HowB112 harboring the Talaromyces emersonii gene was centrifuged at 9000 rpm and dialyzed against 20 mM NaOac, pH 5 over night. The solution was then applied on a S Sepharose column (200 ml) previously equilibrated in 20 mM NaOAc, pH 5. The glucoamylase was collected in the effluent, and applied on a Q Sepharose column (50 ml) previously equilibrated in 20 mM NaOAC, pH 4.5. Unbound material was washed of the column and the glucoamylase was eluted using a linear gradient from 0-0.3 M NaCl in 20 mM NaOAc over 10 column volumes. Purity of the glucoamylase fraction was checked by SDS-PAGE and only one single band was seen. The molecular weight was again found to about 70 kdal as seen for the wild type glucoamylase. The specific activity towards maltose was measured and a specific activity of 8.0 AGU/mg (37°C) and 21.0 AGU/mg (60°C) were found which is in accordance the data on the wild type enzyme.

### EXAMPLE 10

### kinetic Parameters

Kinetic Parameters for Hydrolysis of Maltose and Isomaltose by *Aspergillus niger* AMG and the recombinant *Talaromyces emersonii* AMG expressed in *A. niger*.

| Maltose | k_{cat} (s⁻¹)^{a} | Kₘ (mM) | K_{cat}/Kₘ (s⁻¹mM⁻¹) |
|---|---|---|---|
| *Talaromyces emersonii* | 30.6 | 3.8 | 8.1 |
| *Aspergillus nigger* | 10.7 | 1.2 | 8.8 |

| | | | |
|---|---|---|---|
| ^{a} At 45°C using 0.05 M NaOAc, pH 4.5 | | | |

| Isomaltose | k_{cat} (s⁻¹)^{a} | Kₘ (mM) | K_{cat}/Kₘ (s⁻¹mM⁻¹) |
|---|---|---|---|
| Talaromyces emersonii | 2.70 | 53.6 | 0.050 |
| *Aspergillus niger* | 0.41 | 19.8 | 0.021 |

| | | | |
|---|---|---|---|
| ^{a} At 45°C using 0.05 M NaOAc, pH 4.5 | | | |

### EXAMPLE 11

### Saccharification performance of recombinant Talaromyces emersonii AMG produced in A. nigger

The saccharification performance of the *Talaromyces emersonii* glucoamylase was tested at different temperatures with and without the addition of acid α-amylase and pullulanase. Saccharification was run under the following conditions:
Substrate: 10 DE Maltodextrin, approx. 30% DS (w/w)
Temperatures: 60, 65, or 70°C
Initial (pH: 4.5
Enzyme dosage:
Recombinant *Talaromyces emersonii* glucoamylase produced *in* A. *niger*: 0.24 or 0.32 AGU/g DS
Acid α-amylase derived from *A. niger*: 0.020 AFAU/g DS
Pullulanase derived from *Bacillus*: 0.03 PUN/g DS

When used alone *Talaromyces* AMG was dosed at the high dosage (0.32 AGU/g DS), otherwise at the low dosage, i.e., 0.24 AGU/g DS.

### Saccharification

The substrate for saccharification was made by dissolving maltodextrin (prepared from common corn) in boiling Milli-Q water and adjusting the dry substance to approximately 30% (w/w). pH was adjusted to 4.5 (measured at 60°C). Aliquots of substrate corresponding to 150g dry solids were transferred to 500 ml blue cap glass flasks and placed in a water bath with stirring at the respective temperatures. Enzymes were added and pH readjusted if necessary (measured at incubation temperature). Samples were taken periodically and analysed at HPLC for determination of the carbohydrate composition.

The glucose produced during saccharification are given in the table below, the first three columns representing the saccharification with glucoamylase and acrid α-amylase and pullulanase, the last three with glucoamylase alone. Numbers are % DP1 on DS.

| Time | 0.24 AGU+0.02AFAU+0.03PUN | | | 0.32 AGU | | |
|---|---|---|---|---|---|---|
| (hours) | 60°C | 65°C | 70°C | 60°C | 65°C | 70°C |
| 24 | 88.96 | 90.51 | 87.91 | 84.98 | 86.28 | 84.35 |
| 48 | 94.03 | 94.28 | 9190. | 88.86 | 89.51 | 86.98 |
| 72 | 95.08 | 94.75 | 93.12 | 90.18 | 90.42 | 87.99 |
| 98 | 95.03 | 94.59 | 93.64 | 90.65 | 90.72 | 88.51 |

A glucose yield above 95% was obtained after 72 hours using an enzyme dosage of 0.24 AGU/g DS which is corresponding to 0.03 mg/g DS. The typical dosage of A. nigger AMG would be 0.18 AGU/g DS which is corresponding to 0.09 mg/g, DS to get a yield og 95-96% glucose. A significantly lower enzyme dosage in mg enzyme protein of *Talaromyces*. AMG is therefore required in the saccharification process compared to *A. niger* AMG due to the high specific activity of *T. emersonii* AMG.

### Example 12

### Temperature stability - T½ (half-life) of recombinant Talaromyces emersonii AMG expressed in yeast

The thermal stability of recombinant *Talaromyces emersonii* glucoamylase expressed in yeast (purified using the method described in Example 9) was determined at 70°C, pH 4.5, 0.2 AGU/ml using the method described above in the "Material and Methods" section as "Thermal Stability I (T½ (half-life) determination of AMG".

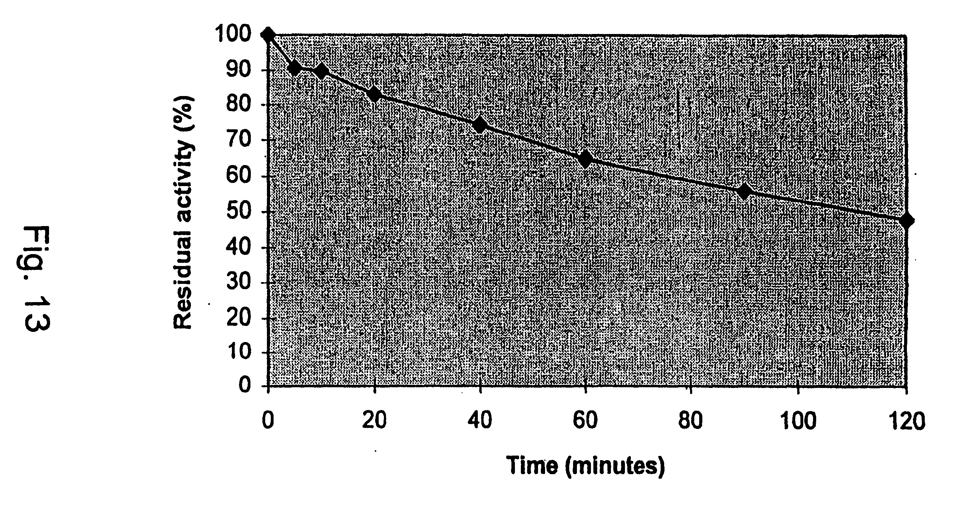

Figure 13 shows the result of the test. The T½ of the Recombinant Talaromyces emersonii glucoamylase expressed in yeast was determined to about 110 minutes at 70°C.

## Claims

1. An Isolated enzyme with glucoamylase activity, wherein the enzyme,
a) is derived from *Talaromyces emersonii,*
b) exhibits a degree of identity of at least 80% with the amino acid sequence shown in SEQ ID NO: 7, and
c) has a T_{1/2} (half-life) of at least 100 minutes in 50 mM NaOAc, 0.2 AGU/ml, pH 4.5, at 70°C.

2. The enzyme according to claim 1, which enzyme has a T_{1/2} (half-life) In the range from 100-140 minutes.

3. The enzyme according to claims 1-2, wherein the *Talaromyces emersonii* is *Talaromyces emersonii* CBS 793.97.

4. A cloned DNA sequence derived from *Talaromyces emersonii* and encoding an enzyme exhibiting glucoamylase activity, which DNA sequence comprises:
(a) the glucoamylase encoding part of the DNA sequence shown In SEQ ID NO: 33;
(b) the DNA sequence shown in positions 649-2724 in SEQ ID NO:33 or its complementary strand;
(c) an analogue of the DNA sequence defined In (a) or (b) which is at least 80% homologous with said DNA sequence;
(d) a DNA sequence which hybridizes with a double-stranded DNA probe comprising the sequence shown in 649-2724 in SEQ ID NO: 33 at medium stringency:
(e) a DNA sequence which, because of the degeneracy of the genetic code, does not hybridize with the sequences of (b) or (c) but which codes for a polypeptide having exactly the same amino add sequence as the polypeptide encoded by any of these DNA sequences.

5. The DNA sequence of claim 4, wherein the DNA sequence is derived from the deposited T. *emersonli* CBS 793.97.

6. A process for converting starch or partially hydrolyzed starch into a syrup containing dextrose, said process including the step of saccharifying starch hydrolyzate in the presence of a glucoamylase according to any of claims 1-3 or a glucoamylase encoded by a DNA sequence of claims 4 or 5.

7. The process 6, of claim wherein the dosage of glucoamylase is present in the range from 0.05 to 0.5 AGU per gram of dry solids.

8. The process of any claims 6 or 7, comprising saccharification of a starch hydrolyzate of at least 30 percent by weight of dry solids.

9. The process of any of claims 6 to 8, wherein the saccharification is conducted in the presence of a debranching enzyme selected from the group of pullulanase and isoamylase, preferably a pullulanase derived from *Bacillus acidopullulyticus* or *Bacillus deramificans* or an isoamylase derived from *Pseudomonas amyloderamosa.*

10. The process of any of claims 6 to 9, wherein the saccharification is conducted at a pH of about 3 to 5.5 and at a temperature of 60-80°C.

11. A method of saccharifying a liquefied starch solution, which method comprises an enzymatic saccharification step using a glucoamylase according to any of claims 1-3 or a glucoamylase encoded by a DNA sequence of claims 4 or 5.

12. Use of a glucoamylase according to any one of claims 1-3 or a glucoamylase encoded by a DNA sequence of claims 4 or 5 in a starch conversion process.

13. Use of a glucoamylase according to any one of claims 1-3 or a glucoamylase encoded by a DNA sequence of claims 4 or 5 in a continuous starch conversion process.

14. Use of a glucoamylase according to any one of claims 1-3 or a glucoamylase encoded by a DNA sequence of claims 4 or 5 in a process for producing oligosaccharides.

15. Use of a glucoamylase according to any one of claims 1-3 or a glucoamylase encoded by a DNA sequence of claims 4 or 5 in a process for producing specialty syrups.

16. Use of a glucoamylase according to any one of claims 1-3 or a glucoamylase encoded by a DNA sequence of claims 4 or 5 in a process for producing ethanol for fuel.

17. Use of a glucoamylase according to any one of claims 1-3 or a glucoamylase encoded by a DNA sequence of claims 4 or 5 in a process for producing a beverage.

18. Use of a glucoamylase according to any one of claims 1-3 or a glucoamylase encoded by a DNA sequence of claims 4 or 5 in a fermentation process for producing organic compounds, such as citric acid, ascorbic acid, lysine, glutamic acid.

19. An isolated pure culture of the microorganism *Talaromyces emersonii* CBS 793.97 capable of producing a glycoamylase as defined in any of the claims 1-3 or a glucoamylase encoded by a DNA sequence of claims 4 or 5.

## Patentansprüche

1. Isoliertes Enzym mit Glucoamylase-Aktivität, wobei das Enzym
(a) von *Talaromyces emersonii* abgeleitet ist,
(b) einen Identitätsgrad von mindestens 80 % mit der in SEQ ID NO:7 gezeigten Aminosäuresequenz aufweist, und
(c) eine T_{1/2} (Halbwertszeit) von mindestens 100 min in 50 mM NaOAc, 0,2 AGU/ml, pH 4,5 bei 70° C aufweist.

2. Enzym nach Anspruch 1, wobei das Enzym eine T_{1/2} (Halbwertszeit) im Bereich von 100 - 140 min aufweist.

3. Enzym nach den Ansprüchen 1-2, wobei der *Talaromyces emersonii Talaromyces emersonii* CBS 793.97 ist.

4. Klonierte DNA-Sequenz, die sich von *Talaromyces emersonii* ableitet und ein Enzym codiert, das Glucoamylase-Aktivität aufweist, wobei die DNA-Sequenz umfasst:
(a) den Glucoamylase-codierenden Teil der in SEQ ID NO:33 gezeigten DNA-Sequenz;
(b) die in den Positionen 649-2724 in SEQ ID NO:33 gezeigte DNA-Sequenz oder ihren Komplementärstrang;
(c) ein Analog der in (a) oder (b) definierten DNA-Sequenz, das zu mindestens 80 % zu der DNA-Sequenz homolog ist;
(d) eine DNA-Sequenz, die mit einer Doppelstrang-DNA-Sonde, die die in 649-2724 in SEQ ID NO:3 gezeigte Sequenz einschließt, bei mittlerer Stringenz hybridisiert;
(e) eine DNA-Sequenz, die aufgrund der Degeneriertheit des generischen Codes nicht mit den Sequenzen (b) oder (c) hybridisiert, sondern die ein Polypeptid mit genau der Aminosäuresequenz wie das Polypeptid, das von einer dieser DNA-Sequenzen codiert wird, codiert.

5. DNA-Sequenz nach Anspruch 4, wobei die DNA-Sequenz von dem hinterlegten *Talaromyces emersonii* CBS 793.97 abgeleitet ist.

6. Verfahren zur Umwandlung von Stärke oder teilweise hydrolysierter Stärke zu einem Sirup, der Dextrose enthält, wobei das Verfahren den Schritt der Saccharifizierung von Stärkehydrolysat in Gegenwart einer Glucoamylase nach einem der Ansprüche 1-3 oder einer Glucoamylase, die von einer DNA-Sequenz der Ansprüche 4 oder 5 codiert wird, einschließt.

7. Verfahren nach Anspruch 6, wobei die Dosis von Glucoamylase im Bereich von 0,05 bis 0,5 AGU pro Gramm trockene Feststoffe liegt.

8. Verfahren nach einem der Ansprüche 6 oder 7, das die Saccharifizierung eines Stärkehydrolysats von mindestens 30 Gew.-% trockener Feststoffe umfasst.

9. Verfahren nach einem der Ansprüche 6 bis 8, wobei die Saccharifizierung in Gegenwart eines Verzweigungen abbauenden Enzyms durchgeführt wird, das aus der Gruppe von Pullulanase und Isoamylase, vorzugsweise einer Pullulanase, die von *Bacillus acidopullulyticus* oder *Bacillus deramificans* abgeleitet ist, oder einer Isoamylase, die von *Pseudomonas amyloderamosa* abgeleitet ist, ausgewählt ist.

10. Verfahren nach einem der Ansprüche 6 bis 9, wobei die Saccharifizierung bei einem pH von etwa 3 bis 5,5 und bei einer Temperatur von 60 - 80° C durchgeführt wird.

11. Verfahren zur Saccharifizierung einer verflüssigten Stärkelösung, wobei das Verfahren einen enzymatischen Saccharifizierungsschritt unter Verwendung einer Glucoamylase nach einem der Ansprüche 1-3 oder einer Glucoamylase, die von einer DNA-Sequenz der Ansprüche 4 oder 5 codiert wird, umfasst.

12. Verwendung einer Glucoamylase nach einem der Ansprüche 1-3 oder einer Glucoamylase, die von einer DNA-Sequenz der Ansprüche 4 oder 5 codiert wird, in einem Stärkeumwandlungsverfahren.

13. Verwendung einer Glucoamylase nach einem der Ansprüche 1-3 oder einer Glucoamylase, die von einer DNA-Sequenz der Ansprüche 4 oder 5 codiert wird, in einem kontinuierlichen Stärkeumwandlungsverfahren.

14. Verwendung einer Glucoamylase nach einem der Ansprüche 1-3 oder einer Glucoamylase, die von einer DNA-Sequenz der Ansprüche 4 oder 5 codiert wird, in einem Verfahren zur Herstellung von Oligosacchariden.

15. Verwendung einer Glucoamylase nach einem der Ansprüche 1-3 oder einer Glucoamylase, die von einer DNA-Sequenz der Ansprüche 4 oder 5 codiert wird, in einem Verfahren zur Herstellung von Spezialsirupen.

16. Verwendung einer Glucoamylase nach einem der Ansprüche 1-3 oder einer Glucoamylase, die von einer DNA-Sequenz der Ansprüche 4 oder 5 codiert wird, in einem Verfahren zur Herstellung von Ethanol für Kraftstoff.

17. Verwendung einer Glucoamylase nach einem der Ansprüche 1-3 oder einer Glucoamylase, die von einer DNA-Sequenz der Ansprüche 4 oder 5 codiert wird, in einem Verfahren zur Herstellung eines Getränks.

18. Verwendung einer Glucoamylase nach einem der Ansprüche 1-3 oder einer Glucoamylase, die von einer DNA-Sequenz der Ansprüche 4 oder 5 codiert wird, in einem Fermentationsverfahren zur Herstellung organischer Verbindungen, wie Citronensäure, Ascorbinsäure, Lysin, Glutaminsäure.

19. Isolierte Reinkultur des Mikroorganismus *Talaromyces emersonii* CBS 793.97, die zur Produktion einer Glucoamylase, wie in einem der Ansprüche 1-3 definiert, oder einer Glucoamylase, die von einer DNA-Sequenz der Ansprüche 4 oder 5 codiert wird, in der Lage ist.

## Revendications

1. Enzyme isolée ayant une activité glucoamylase, dans laquelle l'enzyme
a) est dérivée de *Talaromyces emersonii* ;
b) présente un degré d'identité d'au moins 80 % avec la séquence d'acides aminés montrée dans SEQ ID NO: 7 ; et
c) a un T_{½} (demi-vie) d'au moins 100 minutes dans 50 mM NaOAc, 0,2 AGU/ml, pH 4,5 à 70 °C.

2. Enzyme selon la revendication 1, qui a un T_{½} (demi-vie) dans la gamme de 100-140 minutes.

3. Enzyme selon les revendications 1-2, dans laquelle le *Talaromyces emersonii* est *Talaromyces emersonii* CBS 793.97.

4. Séquence d'ADN clonée dérivée de *Talaromyces emersonii* et codant une enzyme présentant une activité glucoamylase, la séquence d'ADN comprenant :
a) la partie codant une glucoamylase de la séquence d'ADN montrée dans la SEQ ID No 33 ;
b) la séquence d'ADN montrée aux positions 649-2724 dans la SEQ ID No 33 ou son brin complémentaire ;
c) un analogue de la séquence d'ADN définie en (a) ou (b) qui a au moins 80 % d'homologie avec ladite séquence d'ADN ;
d) une séquence d'ADN qui s'hybride avec une sonde double-brin comprenant la séquence montrée en 649-2724 de la SEQ ID No 33 en stringence moyenne ;
e) une séquence d'ADN qui, en raison de la dégénérescence du code génétique, ne s'hybride pas avec les séquences de (b) ou (c), mais qui code pour un polypeptide ayant exactement la même séquence d'acides aminés que le polypeptide codé par l'une quelconque de ces séquences d'ADN.

5. Séquence d'ADN selon la revendication 4, dans laquelle la séquence d'ADN est dérivée de *Talaromyces emersonii* CBS 793.97 déposé.

6. Procédé de conversion de l'amidon ou de l'amidon partiellement hydrolysé en sirop contenant du dextrose, ledit procédé incluant l'étape de saccharification de l'hydrolysat d'amidon en présence d'une glucoamylase selon l'une quelconque des revendications 1-3 ou d'une glucoamylase codée par une séquence d'ADN selon les revendications 4 ou 5.

7. Procédé selon la revendication 6, dans lequel le dosage de la glucoamylase est présent dans la gamme de 0,05 à 0,5 AGU par gramme de matière sèche.

8. Procédé selon l'une quelconque des revendications 6 ou 7, comprenant la saccharification d'un hydrolysat d'amidon d'au moins 30 % en poids de matière sèche.

9. Procédé selon l'une quelconque des revendications 6 à 8, dans lequel la saccharification est effectuée en présence d'une enzyme débranchante sélectionnée dans le groupe d'une pullulanase et d'une isoamylase, de préférence d'une pullulanase dérivée de *Bacillus acidopullulyticus* ou de *Bacillus deramificans* ou une isoamylase dérivée de *Pseudomonas amyloderamosa.*

10. Procédé selon l'une quelconque des revendications 6 à 9, dans lequel la saccharification est effectuée à un pH de 3 à 5,5 et à une température de 60-80°C.

11. Méthode de saccharification d'une solution d'amidon liquéfiée, ladite méthode comprenant une étape de saccharification enzymatique utilisant une glucoamylase selon l'une quelconque des revendications 1-3 ou d'une glucoamylase codée par une séquence d'ADN selon les revendications 4 ou 5.

12. Utilisation d'une glucoamylase selon l'une quelconque des revendications 1-3 ou d'une glucoamylase codée par une séquence d'ADN selon les revendications 4 ou 5 dans un procédé de conversion d'amidon.

13. Utilisation d'une glucoamylase selon l'une quelconque des revendications 1-3 ou d'une glucoamylase codée par une séquence d'ADN selon les revendications 4 ou 5 dans un procédé de conversion d'amidon continu.

14. Utilisation d'une glucoamylase selon l'une quelconque des revendications 1-3 ou d'une glucoamylase codée par une séquence d'ADN selon les revendications 4 ou 5 dans un procédé de production d'oligosaccharides.

15. Utilisation d'une glucoamylase selon l'une quelconque des revendications 1-3 ou d'une glucoamylase codée par une séquence d'ADN selon les revendications 4 ou 5 dans un procédé de production de sirops spécialisés.

16. Utilisation d'une glucoamylase selon l'une quelconque des revendications 1-3 ou d'une glucoamylase codée par une séquence d'ADN selon les revendications 4 ou 5 dans un procédé de production d'éthanol pour carburant.

17. Utilisation d'une glucoamylase selon l'une quelconque des revendications 1-3 ou d'une glucoamylase codée par une séquence d'ADN selon les revendications 4 ou 5 dans un procédé de production d'une boisson.

18. Utilisation d'une glucoamylase selon l'une quelconque des revendications 1-3 ou d'une glucoamylase codée par une séquence d'ADN selon les revendications 4 ou 5 dans un procédé de fermentation pour produire des composés organiques, tels que l'acide citrique, l'acide ascorbique, la lysine, l'acide glutamique.

19. Culture pure isolée du microorganisme *Talaromyces emersonii* CBS 793.97 capable de produire une glucoamylase comme définie dans une quelconque des revendications 1-3 ou une glucoamylase codée par une séquence d'ADN selon les revendications 4 ou 5.


### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): DK 2880
      (G) TELEPHONE: +45 4444 8888
      (H) TELEFAX: +45 4449 3256
   (ii) TITLE OF INVENTION: Thermostable glucoamylase
   (iii) NUMBER OF SEQUENCES: 6
   (iv) COMPUTER READABLE FORM:
      (A) MEDIUM TYPE: Floppy disk
      (B) COMPUTE: IBM PC compatible
      (C) OPERATING SYSTEM: PC-DOS/MS-DOS
      (D) SOFTWARE: Patentin Release #1.0, Version #1.30 (EPO)
(2) INFORMATION FOR SEQ ID NO: 1:
   (i) SEQUENCE CHARACTERISTICS:
      (A) LENGTH: 25 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) STRAIN: Talaromyces emersonii CBS 793.97
   (xi) SEQUENCE DESCRIPTION: SEQ ID NO: 1:
   Ala Asn Gly Ser Leu Asp Ser Phe Leu Ala Thr Glu Xaa Pro Ile Ala
   1               5                   10                  15
   Leu Gln Gly Val Leu Asn Asn Ile Gly
               20                  25
(2) INFORMATION FOR SEQ ID NO: 2:
   (i) SEQUENCE CHARACTERISTICS:
      (A) LENGTH: 20 amino acids
      (B) TYPE: amino acid
      (C) STRANDEDNESS: single
      (D) TOPOLOGY:: linear
   (ii) MOLECULE TYPE: peptide
   (vi) ORIGINAL SOURCE:
      (B) STRAIN: Talaromyces emersonii CBS 793.97
   (xi) SEQUENCE DESCRIPTION: SEQ ID NO: 2:
   Val Gln Thr Ile Ser Asn Pro Ser Gly Asp Leu Ser Thr Gly Gly Leu
   1               5                   10                  15
   Gly Glu Pro Lys
               20
(2) INFORMATION FOR SEQ ID NO: 3:
   (i) SEQUENCE CHARACTERISTICS:
      (A) LENGTH: 22 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) SPRAIN: Talaromyces emersonii CBS 793.97
   (xi) SEQUENCE DESCRIPTION: SEQ ID NO: 3:
   Xaa Asn Val Asn Glu Thr Ala Phe Thr Gly Pro Xaa Gly Arg Pro Gln
   1 5 10 15
   Arg Asp Gly Pro Ala Leu
   20
(2) INFORMATION FOR SEQ ID NO: 4:
   (i) SEQUENCE CHARACTERISTICS:
      (A) LENGTH: 35 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) STRAIN: Talaromyces emersonii CBS 793.97
   (xi) SEQUENCE DESCRIPTION: SEQ ID NO: 4:
   Asp Val Asn Ser Ile Leu Gly Ser Ile His Thr Phe Asp Pro Ala Gly
   1 5 10 15
   Gly Cys Asp Asp Ser Thr Phe Gln Pro Cys Ser Ala Arg Ala Leu Ala
   20 25 30
   Asn His Lys
   35
(2) INFORMATION FOR SEQ ID NO: 5:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) STRAIN: Talaromyces emersonii CBS 793.97
   (xi) SEQUENCE DESCRIPTION: SEQ ID NO: 5:
   Thr Xaa Ala Ala Ala Glu Gln Leu Tyr Asp Ala Ile Tyr Gln Trp Lys
   1 5 10 15
(2) INFORMATION FOR SEQ ID NO: 6:
   (i) SEQUENCE CHARACTERISTICS:
      (A) LENGTH: 35 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) STRAIN: Talaromyces emersonii CBS 793.97
   (xi) SEQUENCE DESCRIPTION: SEQ ID NO: 6:
   Ala Gln Thr Asp Gly Thr Ile Val Trp Glu Asp Asp Pro Asn Arg Ser
   1               5                   10                  15
   Tyr Thr Val Pro Ala Tyr Cys Gly Gln Thr Thr Ala Ile Leu Asp Asp
               20                  25                  30
   Ser Trp Gln
           35
(2) INFORMATION FOR SEQ ID NO: 7:
   (i) SEQUENCE CHARACTERISTICS:
      (A) LENGTH: 591 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (B) STRAIN: Talaromyces emersonii CBS 793.97
   (xi) SEQUENCE DESCRIPTION: SEQ ID NO: 7:
   Ala Thr Gly Ser Leu Asp Ser Phe Leu Ala Thr Glu Thr Pro Ile Ala
   1 5 10 15
   Leu Gln Gly Val Leu Asn Asn Ile Gly Pro Asn Gly Ala Asp Val Ala
   20 25 30
   Gly Ala Ser Ala Gly Ile Val Val Ala Ser Pro Ser Arg Ser Asp Pro
   35 40 45
   Asn Tyr Phe Tyr Ser Trp Thr Arg Asp Ala Ala Leu Thr Ala Lys Tyr
   50 55 60
   Leu Val Asp Ala Phe Asn Arg Gly Asn Lys Asp Leu Glu Gln Thr Ile
   65 70 75 80
   Gln Gln Tyr Ile Ser Ala Gln Ala Lys Val Gln Thr Ile Ser Asn Pro
   85 90 95
   Ser Gly Asp Leu Ser Thr Gly Gly Leu Gly Glu Pro Lys Phe Asn Val
   100 105 110
   Asn Glu Thr Ala Phe Thr Gly Pro Trp Gly Arg Pro Gln Arg Asp Gly
   115 120 125
   Pro Ala Leu Arg Ala Thr Ala Leu Ile Ala Tyr Ala Asn Tyr Leu Ile
   130 135 140
   Asp Asn Gly Glu Ala Ser Thr Ala Asp Glu Ile Ile Trp Pro Ile Val
   145 150 155 160
   Gln Asn Asp Leu Ser Tyr Ile Thr Gln Tyr Trp Asn Ser Ser Thr Phe
   165 170 175
   Asp Leu Trp Glu Glu Val Glu Gly Ser Ser Phe Phe Thr Thr Ala Val
   180 185 190
   Gln His Arg Ala Leu Val Glu Gly Asn Ala Leu Ala Thr Arg Leu Asn
   195 200 205
   His Thr Cys Ser Asn Cys Val Ser Gln Ala Pro Gln Val Leu Cys Phe
   210 215 220
   Leu Gln Ser Tyr Trp Thr Gly Ser Tyr Val Leu Ala Asn Phe Gly Gly
   225 230 235 240
   Ser Gly Arg Ser Gly Lys Asp Val Asn Ser Ile Leu Gly Ser Ile His
   245 250 255
   Thr Phe Asp Pro Ala Gly Gly Cys Asp Asp Ser Thr Phe Gln Pro Cys
   260 265 270
   Ser Ala Arg Ala Leu Ala Asn His Lys Val Val Thr Asp Ser Phe Arg
   275 280 285
   Ser Ile Tyr Ala Ile Asn Ser Gly Ile Ala Glu Gly Ser Ala Val Ala
   290 295 300
   Val Gly Arg Tyr Pro Glu Asp Val Tyr Gln Gly Gly Asn Pro Trp Tyr
   305 310 315 320
   Leu Ala Thr Ala Ala Ala Ala Glu Gln Leu Tyr Asp Ala Ile Tyr Gln
   325 330 335
   Trp Lys Lys Ile Gly Ser Ile Ser Ile Thr Asp Val Ser Leu Pro Phe
   340 345 350
   Phe Gln Asp Ile Tyr Pro Ser Ala Ala Val Gly Thr Tyr Asn Ser Gly
   355 360 365
   Ser Thr Thr Phe Asn Asp Ile Ile Ser Ala Val Gln Thr Tyr Gly Asp
       370                 375                 380
   Gly Tyr Leu Ser Ile Val Glu Lys Tyr Thr Pro Ser Asp Gly Ser Leu
   385                 390                 395                 400
   Thr Glu Gln Phe Ser Arg Thr Asp Gly Thr Pro Leu Ser Ala Ser Ala
                   405                 410                 415
   Leu Thr Trp Ser Tyr Ala Ser Leu Leu Thr Ala Ser Ala Arg Arg Gln
               420                 425                 430
   Ser Val Val Pro Ala Ser Trp Gly Glu Ser Ser Ala Ser Ser Val Leu
           435                 440                 445
   Ala Val Cys Ser Ala Thr Ser Ala Thr Gly Pro Tyr Ser Thr Ala Thr
       450                 455                 460
   Asn Thr Val Trp Pro Ser Ser Gly Ser Gly Ser Ser Thr Thr Thr Ser
   465                 470                 475                 480
   Ser Ala Pro Cys Thr Thr Pro Thr Ser Val Ala Val Thr Phe Asp Glu
                   485                 490                 495
   Ile Val Ser Thr Ser Tyr Gly Glu Thr Ile Tyr Leu Ala Gly Ser Ile
               500                 505                 510
   Pro Glu Leu Gly Asn Trp Ser Thr Ala Ser Ala Ile Pro Leu Arg Ala
           515                 520                 525
   Asp Ala Tyr Thr Asn Ser Asn Pro Leu Trp Tyr Val Thr Val Asn Leu
       530                 535                 540
   Pro Pro Gly Thr Ser Phe Glu Tyr Lys Phe Phe Lys Asn Gln Thr Asp
   545                 550                 555                 560
   Gly Thr Ile Val Trp Glu Asp Asp Pro Asn Arg Ser Tyr Thr Val Pro
                   565                 570                 575
   Ala Tyr Cys Gly Gln Thr Thr Ala Ile Leu Asp Asp Ser Trp Gln
               580                 585                 590
(2) INFORMATION FOR SEQ ID NO: 8:
   (i) SEQUENCE CHARACTERISTICS:
      (A) LENGTH: 1605 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "DNA"
   (vi) ORIGINAL SOURCE:
      (B) STRAIN: Aspergillus niger
   (ix) FEATURE:
      (A) NAME/KEY: sig_peptide
      (B) LOCATION:1..72
   (ix) FEATURE:
      (A) NAME/KEY: may _peptide
      (B) LOCATION:73..1602
   (ix) FEATURE:
      (A) NAME/KEY: CDS
      (B) LOCATION:1..1602
   (xi) SEQUENCE DESCRIPTION: SEQ ID NO: 8:
   ATG TCG TTC CGA TCT CTA CTC GCC CTG AGC GGC CTC GTC TGC ACA GGG          48
   Met Ser Phe Arg Ser Leu Leu Ala Leu Ser Gly Leu Val Cys Thr Gly
   -24             -20                 -15                 -10
   TTG GCA AAT GTG ATT TCC AAG CGC GCG ACC TTG GAT TCA TGG TTG AGC        96
   Leu Ala Asn Val Ile Ser Lys Arg Ala Thr Leu Asp Ser Trp Leu Ser
                -5                   1                   5
   AAC GAA GCG ACC GTG GCT CGT ACT GCC ATC CTG AAT AAC ATC GGG GCG       144
   Asn Glu Ala Thr Val Ala Arg Thr Ala Ile Leu Asn Asn Ile Gly Ala
        10                  15                  20
   GAC GGT GCT TGG GTG TCG GGC GCG GAC TCT GGC ATT GTC GTT GCT AGT       192
   Asp Gly Ala Trp Val Ser Gly Ala Asp Ser Gly Ile Val Val Ala Ser
    25                  30                  35                  40
   CCC AGC ACG GAT AAC CCG GAC TAC TTC TAC ACC TGG ACT CGC GAC TCT       240
   Pro Ser Thr Asp Asn Pro Asp Tyr Phe Tyr Thr Trp Thr Arg Asp Ser
                    45                  50                  55
   GGT CTC GTC CTC AAG ACC CTC GTC GAT CTC TTC CGA AAT GGA GAT ACC       288
   Gly Leu Val Leu Lys Thr Leu Val Asp Leu Phe Arg Asn Gly Asp Thr
                60                  65                  70
   AGT CTC CTC TCC ACC ATT GAG AAC TAC ATC TCC GCC CAG GCA ATT GTC       336
   Ser Leu Leu Ser Thr Ile Glu Asn Tyr Ile Ser Ala Gln Ala Ile Val
            75                  80                  85
   CAG GGT ATC AGT AAC CCC TCT GGT GAT CTG TCC AGC GGC GCT GGT CTC       384
   Gln Gly Ile Ser Asn Pro Ser Gly Asp Leu Ser Ser Gly Ala Gly Leu
        90                  95                 100
   GGT GAA CCC AAG TTC AAT GTC GAT GAG ACT GCC TAC ACT GGT TCT TGG       432
   Gly Glu Pro Lys Phe Asn Val Asp Glu Thr Ala Tyr Thr Gly Ser Trp
   105                 110                 115                 120
   GGA CGG CCG CAG CGA GAT GGT CCG GCT CTG AGA GCA ACT GCT ATG ATC       480
   Gly Arg Pro Gln Arg Asp Gly Pro Ala Leu Arg Ala Thr Ala Met Ile
                   125                 130                 135
   GGC TTC GGG CAG TGG CTG CTT GAC AAT GGC TAC ACC AGC ACC GCA ACG       528
   Gly Phe Gly Gln Trp Leu Leu Asp Asn Gly Tyr Thr Ser Thr Ala Thr
               140                 145                 150
   GAC ATT GTT TGG CCC CTC GTT AGG AAC GAC CTG TCG TAT GTG GCT CAA       576
   Asp Ile Val Trp Pro Leu Val Arg Asn Asp Leu Ser Tyr Val Ala Gln
           155                 160                 165
   TAC TGG AAC CAG ACA GGA TAT GAT CTC TGG GAA GAA GTC AAT GGC TCG       624
   Tyr Trp Asn Gln Thr Gly Tyr Asp Leu Trp Glu Glu Val Asn Gly Ser
       170                 175                 180
   TCT TTC TTT ACG ATT GCT GTG CAA CAC CGC GCC CTT GTC GAA GGT AGT       672
   Ser Phe Phe Thr Ile Ala Val Gln His Arg Ala Leu Val Glu Gly Ser
   185                 190                 195                 200
   GCC TTC GCG ACG GCC GTC GGC TCG TCC TGC TCC TGG TGT GAT TCT CAG       720
   Ala Phe Ala Thr Ala Val Gly Ser Ser Cys Ser Trp Cys Asp Ser Gln
                   205                 210                 215
   GCA CCC GAA ATT CTC TGC TAC CTG CAG TCC TTC TGG ACC GGC AGC TTC       768
   Ala Pro Glu Ile Leu Cys Tyr Leu Gln Ser Phe Trp Thr Gly Ser Phe
               220                 225                 230
   ATT CTG GCC AAC TTC GAT AGC AGC CGT TCC GGC AAG GAC GCA AAC ACC       816
   Ile Leu Ala Asn Phe Asp Ser Ser Arg Ser Gly Lys Asp Ala Asn Thr
           235                 240                 245
   CTC CTG GGA AGC ATC CAC ACC TTT GAT CCT GAG GCC GCA TGC GAC GAC       864
   Leu Leu Gly Ser Ile His Thr Phe Asp Pro Glu Ala Ala Cys Asp Asp
       250                 255                 260
   TCC ACC TTC CAG CCC TGC TCC CCG CGC GCG CTC GCC AAC CAC AAG GAG      912
   Ser Thr Phe Gln Pro Cys Ser Pro Arg Ala Leu Ala Asn His Lys Glu
   265                 270                 275                 280
   GTT GTA GAC TCT TTC CGC TCA ATC TAT ACC CTC AAC GAT GGT CTC AGT      960
   Val Val Asp Ser Phe Arg Ser Ile Tyr Thr Leu Asn Asp Gly Leu Ser
                   285                 290                 295
   GAC AGC GAG GCT GTT GCG GTG GGT CGG TAC CCT GAG GAC ACG TAC TAC     1008
   Asp Ser Glu Ala Val Ala Val Gly Arg Tyr Pro Glu Asp Thr Tyr Tyr
               300                 305                 310
   AAC GGC AAC CCG TGG TTC CTG TGC ACC TTG GCT GCC GCA GAG CAG TTG     1056
   Asn Gly Asn Pro Trp Phe Leu Cys Thr Leu Ala Ala Ala Glu Gln Leu
           315                 320                 325
   TAC GAT GCT CTA TAC CAG TGG GAC AAG CAG GGG TCG TTG GAG GTC ACA     1104
   Tyr Asp Ala Leu Tyr Gln Trp Asp Lys Gln Gly Ser Leu Glu Val Thr
       330                 335                 340
   GAT GTG TCG CTG GAC TTC TTC AAG GCA CTG TAC AGC GAT GCT GCT ACT     1152
   Asp Val Ser Leu Asp Phe Phe Lys Ala Leu Tyr Ser Asp Ala Ala Thr
   345                 350                 355                 360
   GGC ACC TAC TCT TCG TCC AGT TCG ACT TAT AGT AGC ATT GTA GAT GCC     1200
   Gly Thr Tyr Ser Ser Ser Ser Ser Thr Tyr Ser Ser Ile Val Asp Ala
                   365                 370                 375
   GTG AAG ACT TTC GCC GAT GGC TTC GTC TCT ATT GTG GAA ACT CAC GCC     1248
   Val Lys Thr Phe Ala Asp Gly Phe Val Ser Ile Val Glu Thr His Ala
               380                 385                 390
   GCA AGC AAC GGC TCC ATG TCC GAG CAA TAC GAC AAG TCT GAT GGC GAG     1296
   Ala Ser Asn Gly Ser Met Ser Glu Gln Tyr Asp Lys Ser Asp Gly Glu
           395                 400                 405
   CAG CTT TCC GCT CGC GAC CTG ACC TGG TCT TAT GCT GCT CTG CTG ACC     1344
   Gln Leu Ser Ala Arg Asp Leu Thr Trp Ser Tyr Ala Ala Leu Leu Thr
       410                 415                 420
   GCC AAC AAC CGT CGT AAC TCC GTC GTG CCT GCT TCT TGG GGC GAG ACC     1392
   Ala Asn Asn Arg Arg Asn Ser Val Val Pro Ala Ser Trp Gly Glu Thr
   425                 430                 435                 440
   TCT GCC AGC AGC GTG CCC GGC ACC TGT GCG GCC ACA TCT GCC ATT GGT     1440
   Ser Ala Ser Ser Val Pro Gly Thr Cys Ala Ala Thr Ser Ala Ile Gly
                   445                 450                 455
   ACC TAC AGC AGT GTG ACT GTC ACC TCG TGG CCG AGT ATC GTG GCT ACT     1488
   Thr Tyr Ser Ser Val Thr Val Thr Ser Trp Pro Ser Ile Val Ala Thr
               460                 465                 470
   GGC GGC ACC ACT ACG ACG GCT ACC CCC ACT GGA TCC GGC AGC GTG ACC     1536
   Gly Gly Thr Thr Thr Thr Ala Thr Pro Thr Gly Ser Gly Ser Val Thr
           475                 480                 485
   TCG ACC AGC AAG ACC ACC GCG ACT GCT AGC AAG ACC AGC ACC ACG ACC     1584
   Ser Thr Ser Lys Thr Thr Ala Thr Ala Ser Lys Thr Ser Thr Thr Thr
       490                 495                 500
   CGC TCT GGT ATG TCA CTG TGA                                         1605
   Arg Ser Gly Met Ser Leu
   505                 510
(2) INFORMATION FOR SEQ ID NO: 9:
   (i) SEQUENCE CHARACTERISTICS:
      (A) LENGTH: 534 amino acids
      (B) TYPE: amino acid
      (D) TOPOLOGY: linear
   (ii) MOLECULE TYPE: protein
   (xi) SEQUENCE DESCRIPTION: SEQ ID NO: 9:
   Met Ser Phe Arg Ser Leu Leu Ala Leu Ser Gly Leu Val Cys Thr Gly
   -24             -20                 -15                 -10
   Leu Ala Asn Val Ile Ser Lys Arg Ala Thr Leu Asp Ser Trp Leu Ser
                -5                   1                   5
   Asn Glu Ala Thr Val Ala Arg Thr Ala Ile Leu Asn Asn Ile Gly Ala
        10                  15                  20
   Asp Gly Ala Trp Val Ser Gly Ala Asp Ser Gly Ile Val Val Ala Ser
    25                  30                  35                  40
   Pro Ser Thr Asp Asn Pro Asp Tyr Phe Tyr Thr Trp Thr Arg Asp Ser
                    45                  50                  55
   Gly Leu Val Leu Lys Thr Leu Val Asp Leu Phe Arg Asn Gly Asp Thr
                60                  65                  70
   Ser Leu Leu Ser Thr Ile Glu Asn Tyr Ile Ser Ala Gln Ala Ile Val
            75                  80                  85
   Gln Gly Ile Ser Asn Pro Ser Gly Asp Leu Ser Ser Gly Ala Gly Leu
        90                  95                 100
   Gly Glu Pro Lys Phe Asn Val Asp Glu Thr Ala Tyr Thr Gly Ser Trp
   105                 110                 115                 120
   Gly Arg Pro Gln Arg Asp Gly Pro Ala Leu Arg Ala Thr Ala Met Ile
                   125                 130                 135
   Gly Phe Gly Gln Trp Leu Leu Asp Asn Gly Tyr Thr Ser Thr Ala Thr
               140                 145                 150
   Asp Ile Val Trp Pro Leu Val Arg Asn Asp Leu Ser Tyr Val Ala Gln
           155                 160                 165
   Tyr Trp Asn Gln Thr Gly Tyr Asp Leu Trp Glu Glu Val Asn Gly Ser
       170                 175                 180
   Ser Phe Phe Thr Ile Ala Val Gln His Arg Ala Leu Val Glu Gly Ser
   185                 190                 195                 200
   Ala Phe Ala Thr Ala Val Gly Ser Ser Cys Ser Trp Cys Asp Ser Gln
                   205                 210                 215
   Ala Pro Glu Ile Leu Cys Tyr Leu Gln Ser Phe Trp Thr Gly Ser Phe
               220                 225                 230
   Ile Leu Ala Asn Phe Asp Ser Ser Arg Ser Gly Lys Asp Ala Asn Thr
           235                 240                 245
   Leu Leu Gly Ser Ile His Thr Phe Asp Pro Glu Ala Ala Cys Asp Asp
       250                 255                 260
   Ser Thr Phe Gln Pro Cys Ser Pro Arg Ala Leu Ala Asn His Lys Glu
   265                 270                 275                 280
   Val Val Asp Ser Phe Arg Ser Ile Tyr Thr Leu Asn Asp Gly Leu Ser
                   285                 290                 295
   Asp Ser Glu Ala Val Ala Val Gly Arg Tyr Pro Glu Asp Thr Tyr Tyr
               300                 305                 310
   Asn Gly Asn Pro Trp Phe Leu Cys Thr Leu Ala Ala Ala Glu Gln Leu
   315 320 325
   Tyr Asp Ala Leu Tyr Gln Trp Asp Lys Gln Gly Ser Leu Glu Val Thr
   330 335 340
   Asp Val Ser Leu Asp Phe Phe Lys Ala Leu Tyr Ser Asp Ala Ala Thr
   345 350 355 360
   Gly Thr Tyr Ser Ser Ser Ser Ser Thr Tyr Ser Ser Ile Val Asp Ala
   365 370 375
   Val Lys Thr Phe Ala Asp Gly Phe Val Ser Ile Val Glu Thr His Ala
   380 385 390
   Ala Ser Asn Gly Ser Met Ser Glu Gln Tyr Asp Lys Ser Asp Gly Glu
   395 400 405
   Gln Leu Ser Ala Arg Asp Leu Thr Trp Ser Tyr Ala Ala Leu Leu Thr
   410 415 420
   Ala Asn Asn Arg Arg Asn Ser Val Val Pro Ala Ser Trp Gly Glu Thr
   425 430 435 440
   Ser Ala Ser Ser Val Pro Gly Thr Cys Ala Ala Thr Ser Ala Ile Gly
   445 450 455
   Thr Tyr Ser Ser Val Thr Val Thr Ser Trp Pro Ser Ile Val Ala Thr
   460 465 470
   Gly Gly Thr Thr Thr Thr Ala Thr Pro Thr Gly Ser Gly Ser Val Thr
   475 480 485
   Ser Thr Ser Lys Thr Thr Ala Thr Ala Ser Lys Thr Ser Thr Thr Thr
   490 495 500
   Arg Ser Gly Met Ser Leu
   505 510
(2) INFORMATION FOR SEQ ID NO:10:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 102434)"
   (ix) FEATURE:
      (A) NAME/KEY: misc-feature
      (B) LOCATION: 3,6,9,12,15
   (D): OTHER INFORMATION: /Note N= A,G,C or T
      R= G or A
      Y= C or T
      H= A, C or T
   (xi) SEQUENCE DESCRIPTION: SEQ ID NO:10:
      GTNTTRAAYA AYATHGG 17
(2) INFORMATION FOR SEQ ID NO:11:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 102435)"
   (ix) FEATURE:
      (A) NAME/KEY: misc-feature
      (B) LOCATION: 3,6,9,12,15
   (D): OTHER INFORMATION: /Note N= A,G,C or T
      Y= C or T
      H= A, C or T
   (xi) SEQUENCE DESCRIPTION: SEQ ID NO:11:
      GTNCTNAAYA AYATHGG 17
(2) INFORMATION FOR SEQ ID NO:12:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 117360)"
   (ix) FEATURE:
      (A) NAME/KEY: misc-feature
      (B) LOCATION: 3,6,9,12,15
   (D): OTHER INFORMATION: /Note N= A,G,C or T
      R= G or A
      Y= C or T
   (xi) SEQUENCE DESCRIPTION: SEQ ID NO:12:
      CTRGANACCC TYCTYCA 17
(2) INFORMATION FOR SEQ ID NO:13:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 117361)"
   (ix) FEATURE:
      (A) NAME/KEY: mise-feature
      (B) LOCATION: 3,6,12,15
   (D): OTHER INFORMATION: /Note R= G or A
      Y= C or T
   (xi) SEQUENCE DESCRIPTION: SEQ ID NO:13:
      CTRAAYACCC TYCTYCA 17
(2) INFORMATION FOR SEQ ID NO:14:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 127420)"
   (ix) FEATURE:
      (A) NAME/KEY: misc-feature
      (B) LOCATION: 6,9,12,15
   (D): OTHER INFORMATION: /Note N= A,G,C or T
      R= G or A
      Y= C or T
   (xi) SEQUENCE DESCRIPTION: SEQ ID NO:14:
      ACCCTYCTRC TRGGNTT 17
(2) INFORMATION FOR SEQ ID NO:15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 123036"
   (xi) SEQUENCE DESCRIPTION: SEQ ID NO:15:
      GTGAGCCCAA GTTCAATGTG 20
(2) INFORMATION FOR SEQ ID NO:16:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 1"
   (xi) SEQUENCE DESCRIPTION: SEQ ID NO:15:
      AGAAATCGGG TATCCTTTCA G 21
(2) INFORMATION FOR SEQ ID NO:17:
   (i) SEQUENCE CHARACTERISTICS:
      (A) LENGTH: 105 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 2"
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "primer 139746"
   (xi) SEQUENCE DESCRIPTION: SEQ ID NO:18:
      GACAGATCTC CACCATGGCG TCCCTCGTTG 30
(2) INFORMATION: FOR SEQ ID NO:19:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 3"
   (xi) SEQUENCE DESCRIPTION: SEQ ID NO:19:
      GACCTCGAGT CACTGCCAAC TATCGTC 27
(2) INFORMATION FOR SEQ ID NO:20:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "primer 4"
   (xi) SEQUENCE DESCRIPTION: SEQ ID NO:20:
      CCCTCACCAG GGGAATGCTG CAGTTGATG 29
(2) INFORMATION FOR SEQ ID NO:21:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer 950847"
   (xi) SEQUENCE DESCRIPTION: SEQ ID NO:21:
      CGCCATTCTC GGCGACTT 18
(2) INFORMATION FOR SEQ ID NO:22:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "primer 951216"
   (xi) SEQUENCE DESCRIPTION: SEQ ID NO:22:
      CGCCGCGGTA TTCTGCAG 18
(2) INFORMATION FOR SEQ ID NO:23:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 1"
   (xi) SEQUENCE DESCRIPTION: SEQ ID NO:23:
      CAATATAAAC GACGGTACCC GGGAGATCTC CACCATGGCG TCCCTCGTTG 50
(2) INFORMATION FOR SEQ ID NO:24:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc - Tal 4"
   (xi) SEQUENCE DESCRIPTION: SEQ ID NO:24:
      CTAATTACAT CATGCGGCCC TCTAGATCAC TGCCAACTAT CGTC 44
(2) INFORMATION FOR SEQ ID NO:25:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 5"
   (xi) SEQUENCE DESCRIPTION: SEQ ID NO:25:
      AATTTGGGTC GCTCCTGCTC G 21
(2) INFORMATION FOR SEQ ID NO:26:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 6"
   (xi) SEQUENCE DESCRIPTION: SEQ ID NO:26:
      CGAGCAGGAG CGACCCAAAT TATTTCTACT CCTGGACACG 40
(2) INFORMATION FOR SEQ ID NO: 21 :
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 7"
   (xi) SEQUENCE DESCRIPTION: SEQ ID NO:27:
      GATGAGATAG TTCGCATA CG 22
(2) INFORMATION FOR SEQ ID NO:28:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 8"
   (xi) SEQUENCE DESCRIPTION: SEQ ID NO:28:
      CGTATGCGAA CTATCTCATC GACAACGGCG AGGCTTCGAC TGC 43
(2) INFORMATION FOR SEQ ID NO:29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 9"
   (xi) SEQUENCE DESCRIPTION: SEQ ID NO:29:
      CGAAGGTGGA TGAGTTCCAG 29
(2) INFORMATION FOR SEQ ID NO:30:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 10"
   (xi) SEQUENCE DESCRIPTION: SEQ ID NO:30:
      CTGGAACTCA TCCACCTTCG ACCTCTGGGA AGAAGTAGAA GG 42
(2) INFORMATION FOR SEQ ID NO:31:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 11)"
   (xi) SEQUENCE DESCRIPTION: SEQ ID NO:31:
      GACAATACTC AGATATCCAT C 21
(2) INFORMATION FOR SEQ ID NO:32:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Tal 12"
   (xi) SEQUENCE DESCRIPTION: SEQ ID NO:32:
      GATGGATATC TGAGTATTGT CGAGAAATAT ACTCCCTCAG ACG 43
(2) INFORMATION FOR SEQ ID NO: 33:
   (i) SEQUENCE CHARACTERISTICS:
      (A) LENGTH: 2748 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "cDNA"
   (vi) ORIGINAL SOURCE:
      (B) STRAIN: Talaromyces emersonii
   (xi) SEQUENCE DESCRIPTION: SEQ ID NO: 33:
   acgagatgtg tatatactgt gaaccaaact agatgatgtc agttatgctg gtctgagaac 60
   tcatagaagc ccttgaaaat accccaagct agcactccaa ccctaactct gttgctctac 120
   tagatcaaga cgagtactct gattgagctg caggcttgga atatatgatt agcagaaaaa 180
   gggttaaaac ttgtatgaca atcagtttgt cagtactccg tagtgatgcc atgtctatag 240
   agtcgacact aaggcagcat gtgaatgagt cggaaatgac aggaagcaga ttccttaaca 300
   gtcatgttct ccgtgcctgc atccccacgt cacctgcaaa gatgcgacgc tactccacac 360
   cggcgccttg atgtctgctg ttcctggcct agtggagccc catgcgctgc tagctcgtgg 420
   tcttcgaata aatcagaata aaaaacggag taattaattg cgcccgcaac aaactaagca 480
   atgtaactca atgccaagct tccgctgatg ctcttgacat ctccgtagtg gcttctttcg 540
   taatttcaga cgtatatata gtagtaatgc ccagcaggcc gggataatga tggggatttc 600
   tgaactctca gcttccgtac gctgaacagt ttgcttgcgt tgtcaaccat ggcgtccctc 660
   gttgctggcg ctctctgcat cctgggcctg acgcctgctg catttgcacg agcgcccgtt 720
   gcagcgcgag ccaccggttc cctggactcc tttctcgcaa ccgaaactcc aattgccctc 780
   caaggcgtgc tgaacaacat cgggcccaat ggtgctgatg tggcaggagc aagcgccggc 840
   attgtggttg ccagtccgag caggagcgac ccaaattgta ggttctttcc caccagaaat 900
   tacttattta aatcagccct ctgacaggtt gaagatttct actcctggac acgtgacgca 960
   gcgctcacgg ccaaatacct cgtcgacgcc ttcatcgcgg gcaacaagga cctagagcag 1020
   accatccagc agtacatcag cgcgcaggcg aaggtgcaaa ctatctccaa tccgtccgga 1080
   gatttatcca ccggtggctt aggtgagccc aagttcaatg tgaatgagac ggcttttacc 1140
   gggccctggg gtcgtccaca gagggacgga ccagcgttga gagcgacggc cctcattgcg 1200
   tatgcgaact atctcatcgt aagcttctgc tcgctgccct tctctctgct cgtatgctaa 1260
   gtagtcctgt caggacaacg gcgaggcttc gactgccgat gagatcatct ggccgattgt 1320
   ccagaatgat ctgtcctaca tcacccaata ctggaactca tccaccttcg gtaggcaaat 1380
   gaatattccc gacacagcgt ggtactaatt tgattcagac ctctgggaag aagtagaagg 1440
   atcctcattc ttcacaaccg ccgtgcaaca ccgcgccctg gtcgaaggca atgcactggc 1500
   aacaaggctg aaccacacgt gctccaactg cgtctctcag gcccctcagg tcctgtgttt 1560
   cctgcagtca tactggaccg gatcgtatgt tctggccaac tttggtggca gcggtcgttc 1620
   cggcaaggac gtgaattcga ttctgggcag catccacacc tttgatcccg ccggaggctg 1680
   tgacgactcg accttccagc cgtgttcggc ccgtgccttg gcaaatcaca aggtggtcac 1740
   cgactcgttc cggagtatct atgcgatcaa ctcaggcatc gcagagggat ctgccgtggc 1800
   agtcggccgc taccctgagg atgtctacca gggcgggaac ccctggtacc tggccacagc 1860
   agcggctgca gagcagcttt acgacgccat ctaccagtgg aagaagatcg gctcgataag 1920
   tatcacggac gttagtctgc catttttcca ggatatctac ccttctgccg cggtgggcac 1980
   ctataactct ggctccacga ctttcaacga catcatctcg gccgtccaga cgtatggtga 2040
   tggatatctg agtattgtcg tacgttttgc cttagattct caggtgtaaa gaaaaaaatg 2100
   gaactaactc agttctagga gaaatatact ccctcagacg gctctcttac cgaacaattc 2160
   tcccgtacag acggcactcc gctttctgcc tctgccctga cttggtcgta cgcttctctc 2220
   ctaaccgctt cggcccgcag acagtccgtc gtccctgctt cctggggcga aagctccgca 2280
   agcagcgtcc ctgccgtctg ctctgccacc tctgccacgg gcccatacag cacggctacc 2340
   aacaccgtct ggccaagctc tggctctggc agctcaacaa ccaccagtag cgccccatgc 2400
   accactccta cctctgtggc tgtgaccttc gacgaaatcg tcagcaccag ttacggggag 2460
   acaatctacc tggccggctc gatccccgag ctgggcaact ggtccacggc cagcgcgatc 2520
   cccctccgcg cggatgctta caccaacagc aacccgctct ggtacgtgac cgtcaatctg 2580
   ccccctggca ccagcttcga gtacaagttc ttcaagaacc agacggacgg gaccatcgtc 2640
   tgggaagacg acccgaaccg gtcgtacacg gtcccagcgt actgtgggca gactaccgcc 2700
   attcttgacg atagttggca gtgagataac atccaccctt ctgtttta 2748
(2) INFORMATION FOR SEQ ID NO: 34:
   (i) SEQUENCE CHARACTERISTICS:
      (A) LENGTH: 618 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (B) STRAIN: Talaromyces emersonii

      (a) FEATURE:
      (b) NAME/KEY: SIGNAL
      (c) LOCATION: (1) ... (27)
   (xi) SEQUENCE DESCRIPTION: SEQ ID NO: 34:
   Met Ala Ser Leu Val Ala Gly Ala Leu Cys Ile Leu Gly Leu Thr Pro
   1               5                   10                  15
   Ala Ala Phe Ala Arg Ala Pro Val Ala Ala Arg Ala Thr Gly Ser Leu
               20                  25                  30
   Asp Ser Phe Leu Ala Thr Glu Thr Pro Ile Ala Leu Gln Gly Val Leu
           35                  40                  45
   Asn Asn Ile Gly Pro Asn Gly Ala Asp Val Ala Gly Ala Ser Ala Gly
       50                  55                  60
   Ile Val Val Ala Ser Pro Ser Arg Ser Asp Pro Asn Tyr Phe Tyr Ser
   65                  70                  75                  80
   Trp Thr Arg Asp Ala Ala Leu Thr Ala Lys Tyr Leu Val Asp Ala Phe
                   85                  90                  95
   Ile Ala Gly Asn Lys Asp Leu Glu Gln Thr Ile Gln Gln Tyr Ile Ser
               100                 105                 110
   Ala Gln Ala Lys Val Gln Thr Ile Ser Asn Pro Ser Gly Asp Leu Ser
           115                 120                 125
   Thr Gly Gly Leu Gly Glu Pro Lys Phe Asn Val Asn Glu Thr Ala Phe
       130                 135                 140
   Thr Gly Pro Trp Gly Arg Pro Gln Arg Asp Gly Pro Ala Leu Arg Ala
   145                 150                 155                 160
   Thr Ala Leu Ile Ala Tyr Ala Asn Tyr Leu Ile Asp Asn Gly Glu Ala
                   165                 170                 175
   Ser Thr Ala Asp Glu Ile Ile Trp Pro Ile Val Gln Asn Asp Leu Ser
               180                 185                 190
   Tyr Ile Thr Gln Tyr Trp Asn Ser Ser Thr Phe Asp Leu Trp Glu Glu
           195                 200                 205
   Val Glu Gly Ser Ser Phe Phe Thr Thr Ala Val Gln His Arg Ala Leu
       210                 215                 220
   Val Glu Gly Asn Ala Leu Ala Thr Arg Leu Asn His Thr Cys Ser Asn
   225                 230                 235                 240
   Cys Val Ser Gln Ala Pro Gln Val Leu Cys Phe Leu Gln Ser Tyr Trp
                   245                 250                 255
   Thr Gly Ser Tyr Val Leu Ala Asn Phe Gly Gly Ser Gly Arg Ser Gly
               260                 265                 270
   Lys Asp Val Asn Ser Ile Leu Gly Ser Ile His Thr Phe Asp Pro Ala
           275                 280                 285
   Gly Gly Cys Asp Asp Ser Thr Phe Gln Pro Cys Ser Ala Arg Ala Leu
       290                 295                 300
   Ala Asn His Lys Val Val Thr Asp Ser Phe Arg Ser Ile Tyr Ala Ile
   305                 310                 315                 320
   Asn Ser Gly Ile Ala Glu Gly Ser Ala Val Ala Val Gly Arg Tyr Pro
                   325                 330                 335
   Glu Asp Val Tyr Gln Gly Gly Asn Pro Trp Tyr Leu Ala Thr Ala Ala
               340                 345                 350
   Ala Ala Glu Gln Leu Tyr Asp Ala Ile Tyr Gln Trp Lys Lys Ile Gly
           355                 360                 365
   Ser Ile Ser Ile Thr Asp Val Ser Leu Pro Phe Phe Gln Asp Ile Tyr
       370                 375                 380
   Pro Ser Ala Ala Val Gly Thr Tyr Asn Ser Gly Ser Thr Thr Phe Asn
   385                 390                 395                 400
   Asp Ile Ile Ser Ala Val Gln Thr Tyr Gly Asp Gly Tyr Leu Ser Ile
                   405                 410                 415
   Val Glu Lys Tyr Thr Pro Ser Asp Gly Ser Leu Thr Glu Gln Phe Ser
               420                 425                 430
   Arg Thr Asp Gly Thr Pro Leu Ser Ala Ser Ala Leu Thr Trp Ser Tyr
           435                 440                 445
   Ala Ser Leu Leu Thr Ala Ser Ala Arg Arg Gln Ser Val Val Pro Ala
       450                 455                 460
   Ser Trp Gly Glu Ser Ser Ala Ser Ser Val Pro Ala Val Cys Ser Ala
   465                 470                 475                 480
   Thr Ser Ala Thr Gly Pro Tyr Ser Thr Ala Thr Asn Thr Val Trp Pro
                   485                 490                 495
   Ser Ser Gly Ser Gly Ser Ser Thr Thr Thr Ser Ser Ala Pro Cys Thr
               500                 505                 510
   Thr Pro Thr Ser Val Ala Val Thr Phe Asp Glu Ile Val Ser Thr Ser
           515                 520                 525
   Tyr Gly Glu Thr Ile Tyr Leu Ala Gly Ser Ile Pro Glu Leu Gly Asn
       530                 535                 540
   Trp Ser Thr Ala Ser Ala Ile Pro Leu Arg Ala Asp Ala Tyr Thr Asn
   545                 550                 555                     560
   Ser Asn Pro Leu Trp Tyr Val Thr Val Asn Leu Pro Pro Gly Thr Ser
                   565                 570                 575
   Phe Glu Tyr Lys Phe Phe Lys Asn Gln Thr Asp Gly Thr Ile Val Trp
               580                 585                 590
   Glu Asp Asp Pro Asn Arg Ser Tyr Thr Val Pro Ala Tyr Cys Gly Gln
           595                 600                 605
   Thr Thr Ala Ile Leu Asp Asp Ser Trp Gln
       610                 615